# EUROPEAN PATENT APPLICATION

(11) **EP 2 979 706 A1**
(43) Date of publication of application: **03.02.2016**
(21) Application number: 14382298.9
(22) Date of filing: 30.07.2014
(51) Int. Cl.: A61K 45/06, A61K 31/706, A61P 35/00, C12Q 1/68, G01N 33/574

(54) **Methods for treating liver or pancreatic cancer**

(71) Applicant: Fundación Centro Nacional De Investigaciones Oncológicas Carlos III, 28029 Madrid (ES)
(72) Inventor: Djouder, Nabil, E-28029 Madrid (ES); Tummala, Krishna Seshu, E-28029 Madrid (ES)
(74) Representative: ABG Patentes, S.L.

(57) **Abstract**

The invention relates to nicotinamide riboside or a derivative thereof for use in the treatment and/or prevention of liver or pancreatic cancer in a subject. Additionally, the invention relates to an *in vitro* method for designing a customized therapy for a subject diagnosed with a liver cancer or pancreatic cancer or suffering early signs of chronic liver damage or pancreatic intraepithelial lesions based on determining the level of DNA damage. Additionally the invention also relates to a method for diagnosing liver cancer and for predicting the risk of developing liver cancer in a subject suffering hepatitis. The invention also relates to a kit and their use in the methods of the invention.

SEQUENCE LISTING

<110>  FUNDACIÓN CENTRO NACIONAL DE INVESTIGACIONES ONCOLÓGICAS
       CARLOS III

<120>  METHODS FOR TREATING CANCER

<130>  P11012EP00

<160>  40

<170>  PatentIn version 3.5

<210>  1
<211>  24
<212>  DNA
<213>  Artificial Sequence

<220>
<223>  P1-TDO2-F

<400>  1
aggagactac actactggga gaag                                              24


<210>  2
<211>  20
<212>  DNA
<213>  Artificial Sequence

<220>
<223>  P1-TDO2-R

<400>  2
cttgtttgct agcagggcgt                                                   20


<210>  3
<211>  20
<212>  DNA
<213>  Artificial Sequence

<220>
<223>  P4-TDO2-F

<400>  3
ttgtctatgg gcagggtgat                                                   20


<210>  4
<211>  19
<212>  DNA
<213>  Artificial Sequence

<220>
<223>   P4- TDO2-R

<400>  4
gcctggtgcg aaaaacgag                                                    19


<210>  5
<211>  22

<212>  DNA
<213>  Artificial Sequence

<220>
<223>  P5-TDO2-F

<400>  5
ctcctgtaag gacctaccta gc                                                22


<210>  6
<211>  21
<212>  DNA
<213>  Artificial Sequence

<220>
<223>  P5-TDO2-R

<400>  6
accaaactcc tctggcgtat c                                                 21


<210>  7
<211>  21
<212>  DNA
<213>  Artificial Sequence

<220>
<223>  P6-AFMID-F

<400>  7
ctagaggcta gcagcagtgt g                                                 21


<210>  8
<211>  20
<212>  DNA
<213>  Artificial Sequence

<220>
<223>  P6-AFMIDR

<400>  8
cttccagatc gcccagtaca                                                   20


<210>  9
<211>  19
<212>  DNA
<213>  Artificial Sequence

<220>
<223>  P7-AFMID-F

<400>  9
caggcacagt tggatctct                                                    19


<210>  10
<211>  20
<212>  DNA
<213>  Artificial Sequence

<220>
<223>  P7- AFMID-R

<400>  10
agaggacagc ctcgggttat                                                   20


<210>  11
<211>  20
<212>  DNA
<213>  Artificial Sequence

<220>
<223>  P8-AFMID-F

<400>  11
gcgggtgtat ggttacctgt                                                   20


<210>  12
<211>  20
<212>  DNA
<213>  Artificial Sequence

<220>
<223>  P8-AFMID-R

<400>  12
atgcccgagt gttgtgttgt                                                   20


<210>  13
<211>  23
<212>  DNA
<213>  Artificial Sequence

<220>
<223>  P9- AFMID-F

<400>  13
gcctcagtaa atgggttgag aga                                               23


<210>  14
<211>  20
<212>  DNA
<213>  Artificial Sequence

<220>
<223>  P9-AFMID-R

<400>  14
ctgcacgcta cttgcttgtc                                                   20


<210>  15
<211>  20
<212>  DNA
<213>  Artificial Sequence

<220>
<223>  P10-AFMID-F

<400>  15
cactgaggcc aaggctagag                                                   20


<210>  16
<211>  20
<212>  DNA
<213>  Artificial Sequence

<220>
<223>  P10-AFMID-R

<400>  16
aggaacacca tggacgcatt                                                   20


<210>  17
<211>  20
<212>  DNA
<213>  Artificial Sequence

<220>
<223>  P11-AFMID-F

<400>  17
taaaagcagc ccaatgcggg                                                   20


<210>  18
<211>  21
<212>  DNA
<213>  Artificial Sequence

<220>
<223>  P11-AFMID-R

<400>  18
gtcgacctct gacctccaca t                                                 21


<210>  19
<211>  20
<212>  DNA
<213>  Artificial Sequence

<220>
<223>  Chr3-intron-F

<400>  19
cttggccctt cctctcctaa                                                   20


<210>  20
<211>  21
<212>  DNA
<213>  Artificial Sequence

<220>
<223>  Chr3-intron-R

<400>  20
tgcagtggtc agaagatgtg t                                                 21

<210>  21
<211>  20
<212>  DNA
<213>  Artificial Sequence

<220>
<223>  P1-F

<400>  21
gcacagcatt gcggacatgc                                                   20


<210>  22
<211>  20
<212>  DNA
<213>  Artificial Sequence

<220>
<223>  P2-R

<400>  22
ccctccatgt gtgaccaagg                                                   20


<210>  23
<211>  20
<212>  DNA
<213>  Artificial Sequence

<220>
<223>  P3-R

<400>  23
gcagaagcgc ggccgtctgg                                                   20


<210>  24
<211>  18
<212>  DNA
<213>  Artificial Sequence

<220>
<223>  P1-F

<400>  24
tctgagcatg gcctctaa                                                     18


<210>  25
<211>  21
<212>  DNA
<213>  Artificial Sequence

<220>
<223>  P2-R

<400>  25
gctggagtaa atttcacagt g                                                 21


<210>  26
<211>  18

<212>  DNA
<213>  Artificial Sequence

<220>
<223>  P3-R

<400>  26
tctcactcgg aaggacat                                                    18


<210>  27
<211>  20
<212>  DNA
<213>  Artificial Sequence

<220>
<223>   beta Actin-F

<400>  27
cacagctgag agggaaatcg                                                  20


<210>  28
<211>  20
<212>  DNA
<213>  Artificial Sequence

<220>
<223>  beta   Actin-R

<400>  28
agtttcatgg atgccacagg                                                  20


<210>  29
<211>  20
<212>  DNA
<213>  Artificial Sequence

<220>
<223>  Timp1-F

<400>  29
aggtggtctc gttgattcgt                                                  20


<210>  30
<211>  20
<212>  DNA
<213>  Artificial Sequence

<220>
<223>  Timp1-R

<400>  30
gtaaggcctg tagctgtgcc                                                  20


<210>  31
<211>  20
<212>  DNA
<213>  Artificial Sequence

<220>
<223>  alpha -SMA-F

<400>  31
caatggctct gggctctgta                                                   20


<210>  32
<211>  20
<212>  DNA
<213>  Artificial Sequence

<220>
<223>  alpha-SMA-R

<400>  32
tcatccccca catagctgtc                                                   20


<210>  33
<211>  23
<212>  DNA
<213>  Artificial Sequence

<220>
<223>  Col1a-F

<400>  33
agggccaggg ggtccagcat ttc                                               23


<210>  34
<211>  18
<212>  DNA
<213>  Artificial Sequence

<220>
<223>  Col1a1- R

<400>  34
ggtgcccccg gtcttcag                                                     18


<210>  35
<211>  18
<212>  DNA
<213>  Artificial Sequence

<220>
<223>  Bax-F

<400>  35
gtgagcggct gcttgtct                                                     18


<210>  36
<211>  20
<212>  DNA
<213>  Artificial Sequence

<220>
<223>  Bax-R

<400>  36
ggtcccgaag taggagagga                                                    20


<210>  37
<211>  21
<212>  DNA
<213>  Artificial Sequence

<220>
<223>  XIAP-F

<400>  37
gaggcaggaa gctaacgttt t                                                  21


<210>  38
<211>  21
<212>  DNA
<213>  Artificial Sequence

<220>
<223>  XIAP-R

<400>  38
gcagctcgtc tgagatcaat g                                                  21


<210>  39
<211>  20
<212>  DNA
<213>  Artificial Sequence

<220>
<223>  PUMA-F

<400>  39
ctgtatcctg cagcctttgc                                                    20


<210>  40
<211>  19
<212>  DNA
<213>  Artificial Sequence

<220>
<223>  PUMA-R

<400>  40
acgggcgact ctaagtgct                                                     19

## Description

### TECHNICAL FIELD OF THE INVENTION

The invention is related to the field of treatment and diagnostic methods.

### BACKGROUND OF THE INVENTION

Among cancers, liver cancer is known as one of the most aggressive cancers in the world. Liver cancer can be largely classified into hepatocellular carcinoma which arises from hepatocytes.

Hepatocellular carcinoma (HCC) is the commonest, at least 90% of liver cancer, usually lethal, human primary liver neoplasm. The early stage is characterized by low- to high-grade dysplastic nodules, "preneoplastic lesions". These frequently develop in chronic inflammatory liver disease or hepatitis, which can promote fibrosis, cirrhosis and progression to HCC. Thus, precancerous lesions have clinical value for HCC prediction, but therapeutic options are limited.

In early stages of HCC, oncogene activation induces replicative stress, resulting in DNA damage leading to chromosomal instability which accelerates tumor development from preneoplastic lesions. DNA damage elicits a key repair mechanism, the DNA damage response, initiated by phosphorylation of checkpoint proteins Chk1 and Chk2. This induces p53-dependent responses, including cell cycle arrest, apoptosis and/or senescence, which limit preneoplastic lesions' growth, that become cancerous, when p53 function is inactivated. Although oncogene-induced replicative stress is a prerequisite for cancer development, it remains unclear whether it is solely caused through the oncogenic pro-proliferative potential.

HCC has a low survival rate as the majority of patients present with advanced and late stage disease. Coupled to the fact that its aetiology is driven by serious underlying liver disease, HCC patients often have poor performance status and significantly impaired general health, making them less suitable candidates for conventional cancer treatment.

There are three therapeutic mainstays used in the multidisciplinary management of cancer: surgery, radiotherapy and chemotherapy. However HCC has limited therapeutic options.

HCC has always been considered a therapeutic challenge, given the cytotoxic drug resistant nature of the cancer and associated disorder in liver function, reducing the safety of many conventional chemotherapy agents.

Transarterial chemoembolization has become more widely used in the treatment of unresectable HCC (M.C. Chern et al., Cardiovasc. Intervent. Radiol. 31 (2008) 735-744). This treatment combines antineoplastic agents mixed with iodised oil administered via the hepatic artery in an attempt to reduce the rate of tumour growth.

Furthermore, the high frequency of tumor recurrence after curative resection reduces the overall survival of HCC patients. Despite multiple, phase II trials, there are no, well powered, definitive studies which show a survival advantage for chemotherapy. The multikinase inhibitor sorafenib has been found to prolong survival in patients with advanced HCC, by only around 2 months compared to placebo.

Since NAD is important in modulating ADP-ribose polymer metabolism, cyclic ADP-ribose synthesis, and DNA response pathway implicating p53 and following DNA damage, a variety of new pharmaceutical and nutraceutical "NAD+ compounds" have been proposed as an alternative treatment of liver cancer.

Nicotinamide effectively inhibits hepatocarcinogenesis both *in vitro* and *in vivo,* showing lower formation of loci and adenomas (Park SY. et al., J Cell Physiol. 2012 Mar;227(3):899-908). However, the efficacy of NAD+ boosters in the treatment of liver cancer has been questioned, because other studies showed that nicotinic acid or nicotinamide had no effect in the number of foci, their diameter or the proportion of liver occupied by loci in a model of hepatic carcinogenesis (Jackson TM et al., J Nutr. 1995 Jun;125(6):1455-61).

Therefore, alternative treatment strategies with improved therapeutic safety and efficacy that prevent the development and recurrence of liver cancer are urgently needed.

### SUMMARY OF THE INVENTION

In a first aspect, the invention relates to nicotinamide riboside or a derivative thereof for use in the treatment and/or prevention of liver cancer or pancreatic cancer in a subject.

In a second aspect, the invention relates to an *in vitro* method for designing a customized therapy for a subject diagnosed with a liver cancer or pancreatic cancer, suffering early signs of chronic liver damage or suffering pancreatic intraepithelial lesions which comprises
a) determining the level of DNA damage in a sample from said subject, and
b) comparing said level with a reference value,
wherein an increased level of DNA damage in said sample with respect to the reference value is indicative that the subject is to be treated with nicotinamide riboside or a derivative thereof.

In a third aspect, the invention relates to an *in vitro* method for diagnosing liver cancer in a subject which comprises:
a) determining the level of expression of URI gene in a sample from said subject, and
b) comparing said level with a reference value,
wherein an increased level of expression of URI gene in said sample with respect to the reference value is indicative that the subject suffers liver cancer.

In a fourth aspect, the invention relates to an *in vitro* method for predicting the risk of developing liver cancer in a subject suffering hepatitis which comprises:
a) determining the level of expression of URI gene in a sample from said subject, and
b) comparing said levels with a reference value,
wherein an increased level of expression of URI gene in said sample with respect to the reference value is indicative that the subject shows high risk of developing liver cancer.

In a fifth aspect, the invention relates to a kit comprising a reagent which allows determining the expression level of URI gene.

In a sixth seventh aspect, the invention relates to the use of a kit of the invention for designing a customized therapy for a subject diagnosed with a liver cancer or suffering early signs of chronic liver damage, for diagnosing liver cancer or for predicting the risk of a patient suffering hepatitis to develop liver cancer.

### BRIEF DESCRIPTION OF THE DRAWINGS

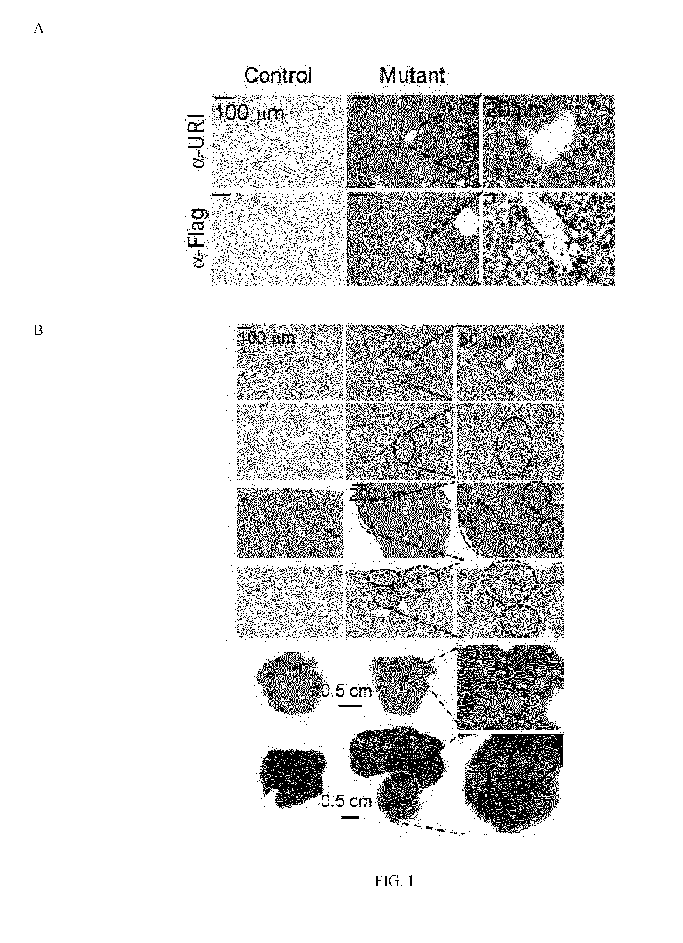

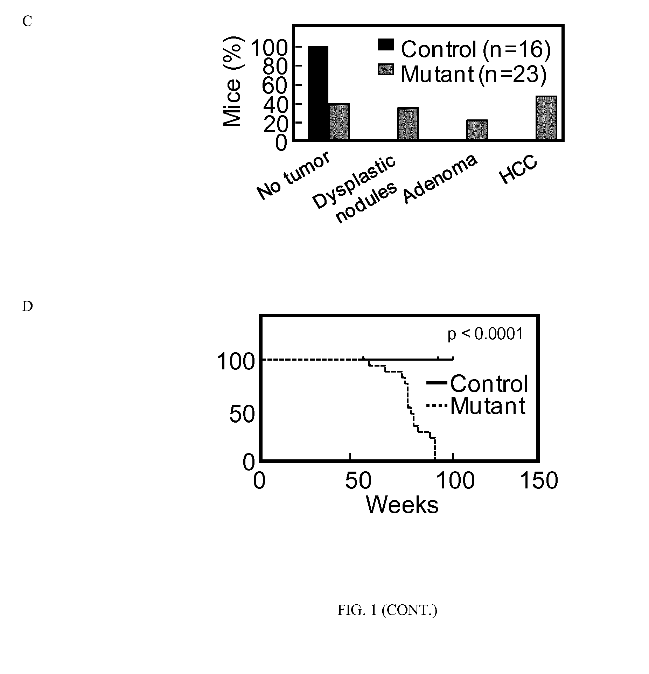

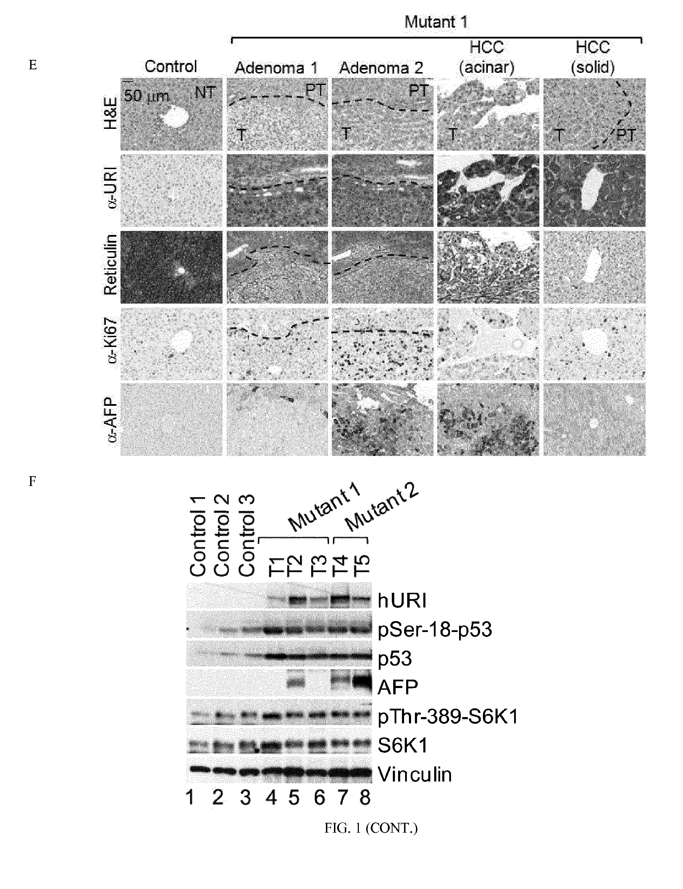

**Figure 1****. URI Expression in Hepatocytes Induces Spontaneous Liver Tumors and Recapitulates Human HCC.** (A) Representative pictures of IHC (immunohistochemistry) stained liver sections from control and mutant hURI-tetOFFhep mice using hURI and FLAG antibodies. Insets represent the periportal area, showing hepatocyte specific hURI expression. n > 10. (B) Representative pictures of H&E (Hematoxylin and eosin) stained liver sections from 3 weeks (n > 6), 8 weeks (n > 19), 12 weeks (n > 11) and for 32 weeks (n > 7) control and mutant hURI-tetOFFhep mice. Bottom pictures are representative images of whole livers from control and mutant hURI-tetOFFhep mice at 32 and 75 weeks of age. Black dotted circles mark anisokaryotic clusters and LLCD-like lesions in mutant hURI-tetOFFhep mice. Yellow dotted circles depict adenoma and HCC at 32 and 75 weeks of age, respectively. (C) Percentage of control (n = 16) and mutant (n = 23) hURI-tetOFFhep mice bearing liver abnormalities in 65 to 75 week old mice. (D) Kaplan Meier curve of control (n = 17) and mutant (n = 17) hURI-tetOFFhep mice. p < 0.0001. (E) Representative pictures of H&E and reticulin stained liver sections from control and 4 tumors derived from 1 mutant hURI-tetOFFhep. IHC was performed using the indicated antibodies. n = 5. (F) WB analysis of control (controls 1 to 3) and mutant (mutants 1 and 2) hURI-tetOFFhep mice. Scale bars represent 20, 50, 100, 200 µm and 0.5 cm, as indicated.

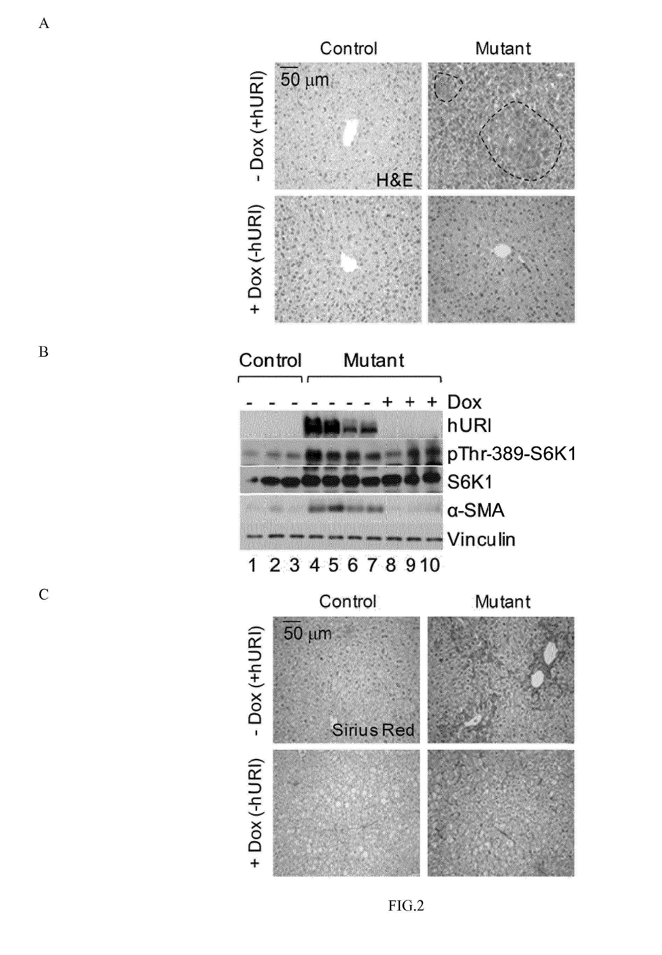

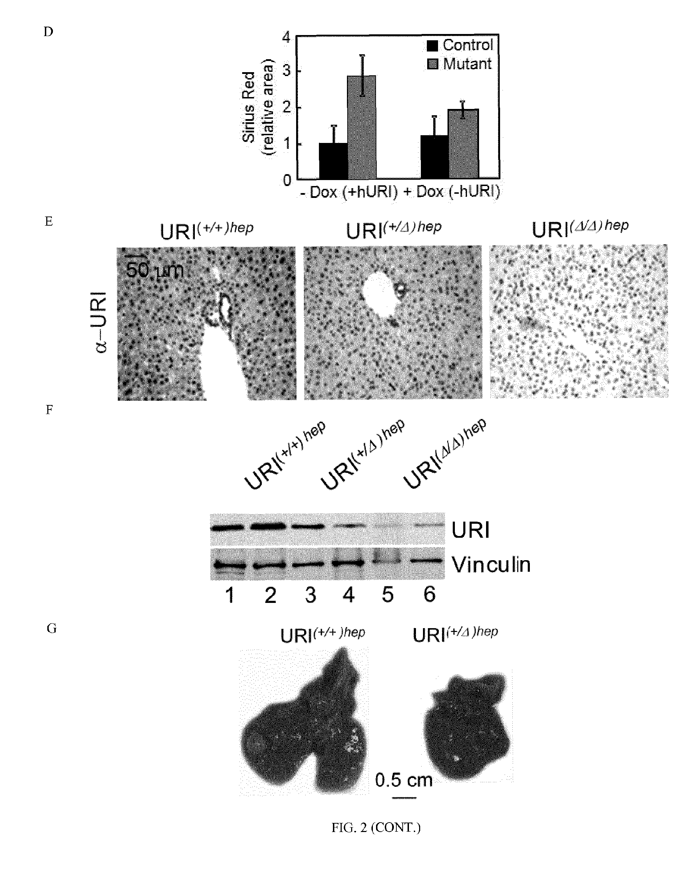

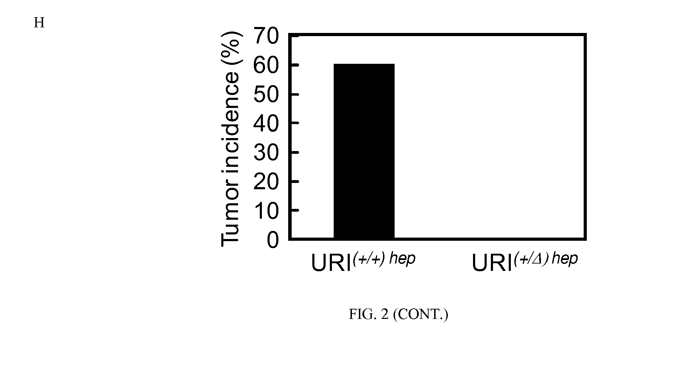

**Figure 2****. URI is Oncogenic and Essential for Hepatocarcinogenesis. (A) Representative pictures** of H&E stained liver sections from 32 week old control and mutant hURI-tetOFFhep mice fed with either chow or doxycycline (Dox) containing-diet. Dotted black circle represents hURI-induced premalignant lesions. Switching off hURI expression with Dox rescues the formation of premalignant clusters. (n ≥ 5). (B) WB analysis of hURI-tetOFFhep mice described in (A). (C) Representative pictures of Sirius Red stained liver sections from hURI-tetOFFhep mice described in (A). Switching off hURI expression with Dox rescues the formation of fibrotic areas. (n ≥ 5). (D) Quantification of (C). (*, p ≤ 0.05). (E) Representative pictures of IHC stained liver sections for endogenous URI in URI(+/+)*hep*, URI(+/Δ)*hep* and URI(Δ/Δ)*hep* mice, confirming hepatocyte-specific reduction or complete loss of URI expression in URI(+/Δ)*hep* and URI(Δ/Δ)*hep* mice, respectively. (n ≥ 3). (F) WB liver analysis for endogenous URI in URI(+/+)*hep*, URI(+/Δ)*hep* and URI(Δ/Δ)*hep* mice, confirming reduction of URI expression in URI(+/Δ)*hep* and URI(Δ/Δ)*hep* mice. (G) Representative images of whole livers from URI(+/+)*hep* and URI(+/Δ)*hep* mice treated with DEN and sacrificed at 24 weeks of age. Dotted yellow circles depict liver tumors present in the URI(+/+)*hep* mice treated with DEN, and which are absence in the URI(+/Δ)*hep* mice. (n ≥ 5). (H) Percentage of tumor incidence in URI(+/+)*hep* and URI(+/Δ)*hep* mice treated with DEN and sacrificed at 24 weeks of age. Reduced levels of URI expression in the URI(+/Δ)*hep* mice confer resistance to DEN-induced tumor formation. (n ≥ 5). Scale bar represents 50 µm and 0.5 cm, as indicated.

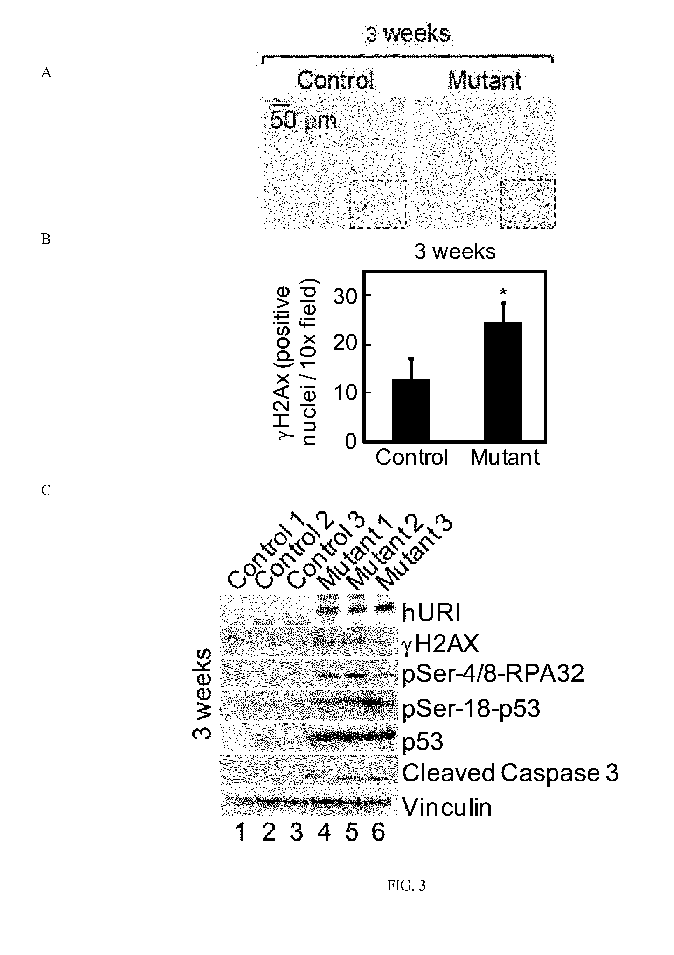

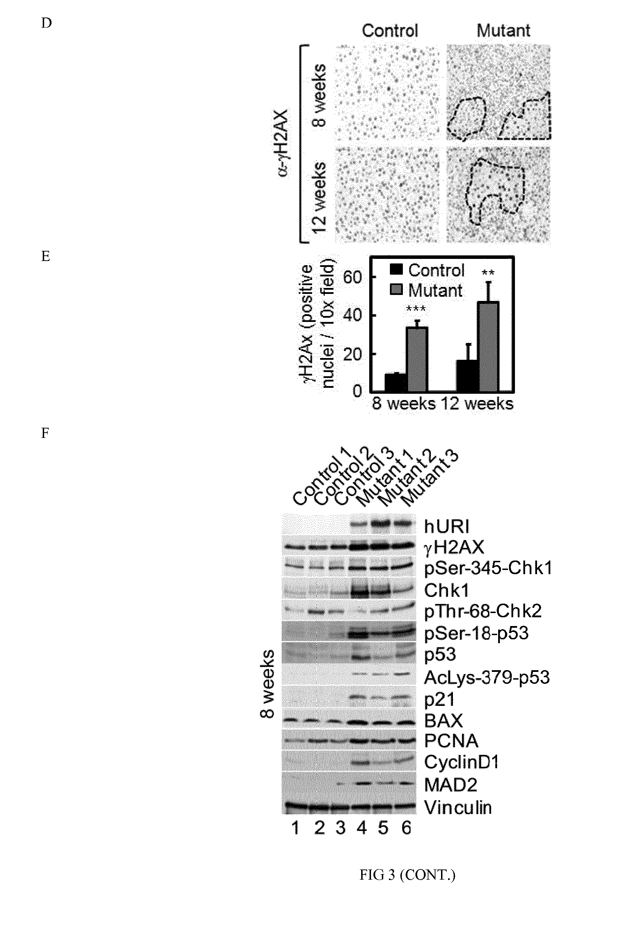

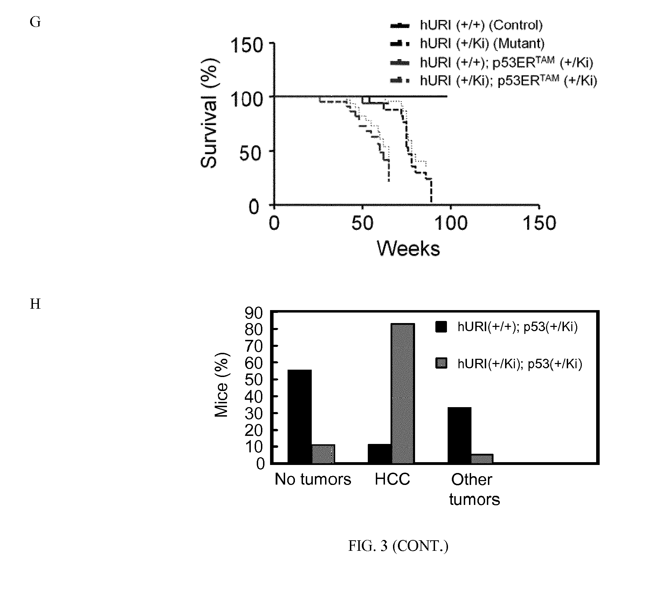

**Figure 3****. URI-Induced DNA Damage before Precancerous Lesion Formation.** (A) Representative pictures of γH2AX IHC stained liver sections from 3 week control and mutant hURI-tetOFFhep mice. Insets denote γH2AX positive nuclei. n = 5. (B) Quantification of (A). (*, p ≤ 0.05). (C) WB analysis of 3 week control and mutant hURI-tetOFFhep mice. (D) Representative pictures of γH2AX IHC stained liver sections from 8 week and 12 week control and mutant hURI-tetOFFhep mice. Dotted black shapes depict anisokaryotic clusters positive for γH2AX. n = 6. (E) Quantification of (D). (**, ρ ≤ 0.01; ***, ρ ≤ 0.001). (F) WB analysis of 8 week control and mutant hURI-tetOFFhep mice. (G) Kaplan-Meier survival curve of control (full line) and mutant (dotted line) hURI-tetOFFhep mice with (red) and without (black) p53 inactivation. Denote that p53 inactivation decreases overall survival in mutant hURI-tetOFFhep mice (dotted red line). (***, ρ ≤ 0.001). Control hURI (+/+) (n = 16), Mutant hURI (+/Ki) (n = 17), hURI (+/+); p53ERTAM (+/Ki) (n = 9), hURI (+/Ki); p53ERTAM (+/Ki) (n = 18). (H) Percentage of tumor incidence in mice described in (G).

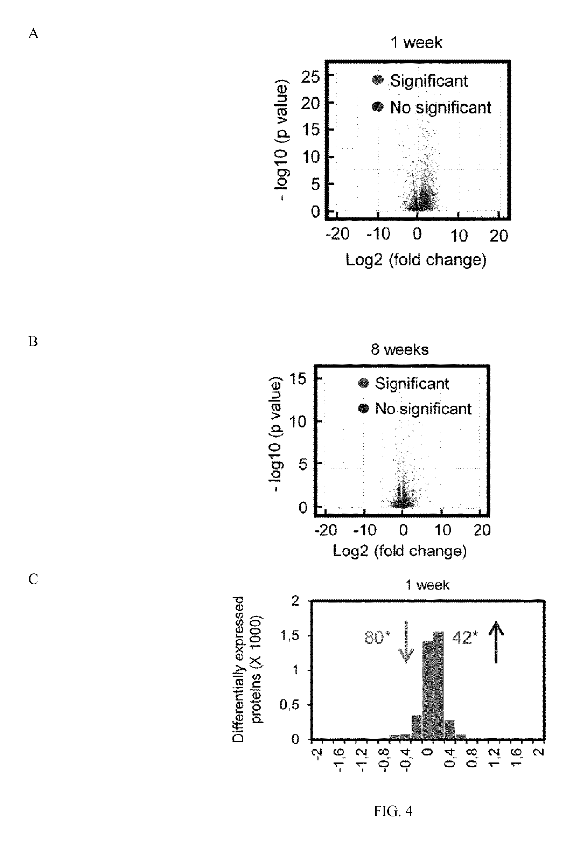

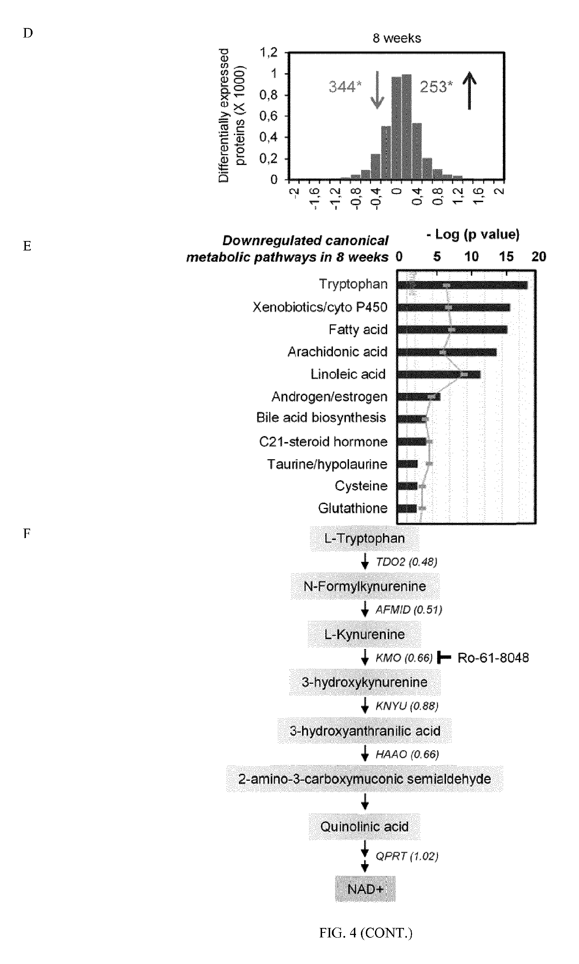

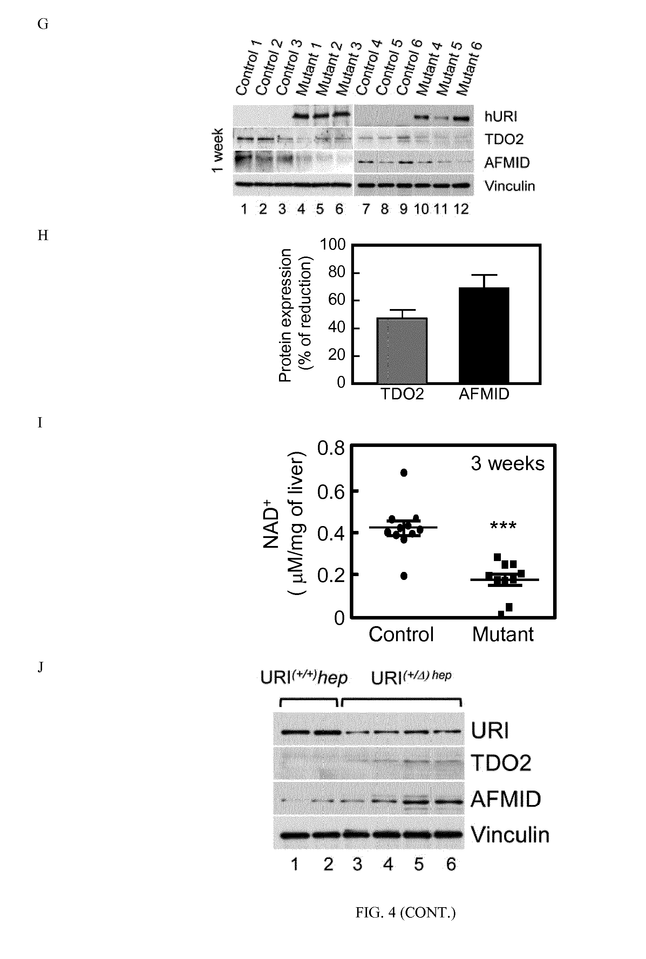

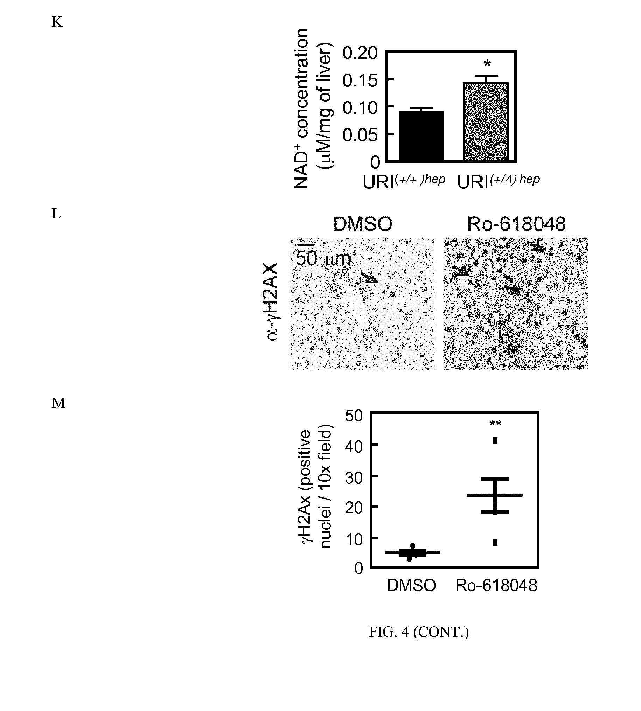

**Figure 4****. URI Causes DNA Damage by Inhibiting *De Novo* NAD+ Synthesis.** (A and B) Volcano plots from RNA sequencing representing significant differentially expressed (blue) and unchanged (red) mRNA species in livers from 1 (A) and 8 (B) week old hURI-tetOFFhep mice. n > 3. (C and D) Histogram representation of differentially expressed proteins by iTRAQ analysis in livers from 1 (C) and 8 (D) week old hURI-tetOFFhep mice. In green are the number of proteins significantly downregulated and in red the number of proteins significantly upregulated. n = 5. (E) Top downregulated canonical metabolic pathways in 8 week iTRAQ data, analyzed by using Ingenuity Pathway Analysis (IPA) software. At this age, the tryptophan pathway (in red) is the top downregulated pathway. (F) Schematic representation of *de novo* NAD+ synthesis. Fold change of protein detected in iTRAQ (mutant over control) are represented within the brackets. Ro-61-8048 is a compound known to block this pathway by inhibiting the activity of kynurenine 3-monooxygenase (KMO). (G) WB analysis of 1 week control and mutant hURI-tetOFFhep mice. (H) Quantification of (G) in terms of percentage of enzyme reduction (mutant over control). (I) Liver NAD+ concentrations in 3 week old control and mutant hURI-tetOFFhep mice. (***, p ≤ 0.001). n ≥ 10. (J) WB analysis of livers from URI(+/+)*hep* and URI(+/Δ)*hep* mice. (*, p ≤ 0.05). (K) NAD+ levels in livers from URI(+/+)*hep* and URI(+/Δ)*hep* mice. (*, ρ ≤ 0.05). n = 5. (L) Representative pictures of γH2AX IHC stained liver sections from C57BL/6 mice previously fed with DDC and treated with either DMSO (1%) or Ro-618048 (25mg/Kg) compound. n = 5. (M) Quantification of (L). (**, ρ ≤ 0.01).

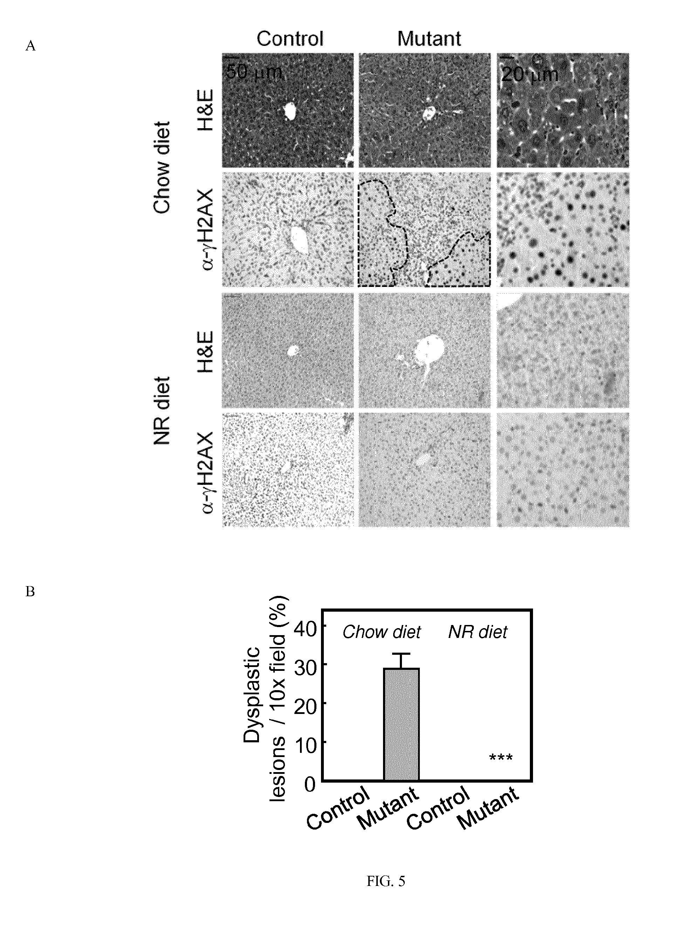

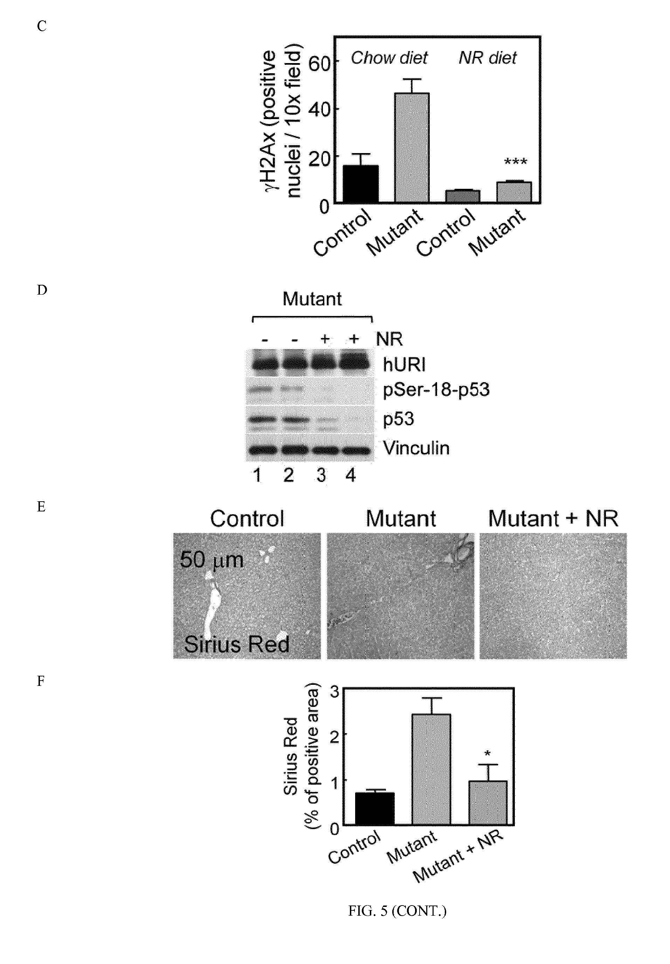

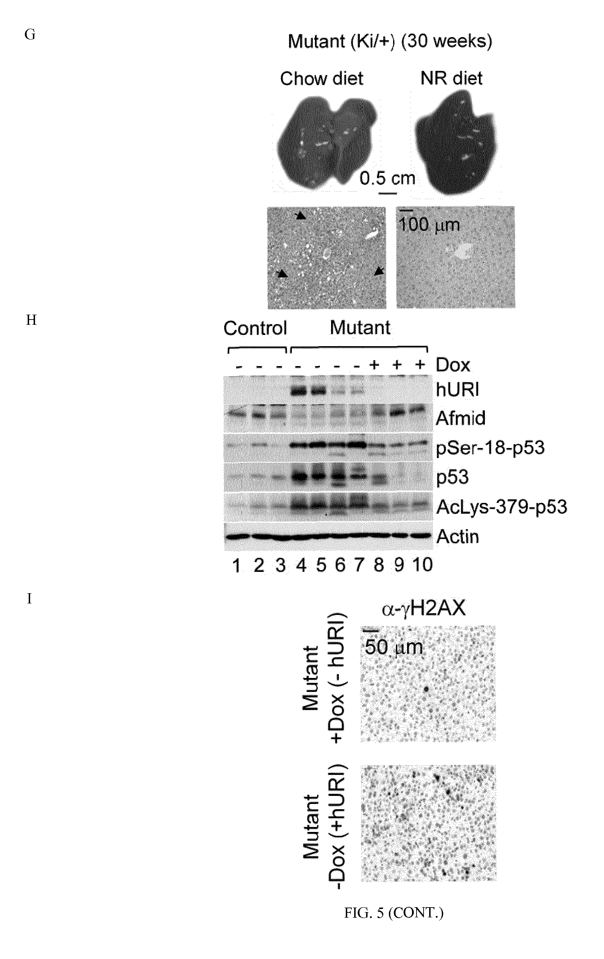

**Figure 5****. Restoring NAD+ Pools Protects from DNA Damage and Aberrant** *De* ***Novo* NAD+ Synthesis Is a Feature of Oncogenesis.** (A) Representative pictures of H&E and γH2AX IHC stained liver sections from 12 week old control and mutant hURI-tetOFFhep mice fed with chow (n = 5) or nicotinamide riboside (NR) diets (n = 7). Dotted black shapes depict anisokaryotic clusters present in mutant hURI-tetOFFhep mice under chow diet. These clusters are no longer present when these mutant hURI-tetOFFhep mice are fed NR diet (lower panels). (B) Quantification of total dysplastic area per slide in the hURI-tetOFFhep mice described in (A). (***, ρ ≤ 0.001). (C) Quantification of γH2AX positive nuclei in the hURI-tetOFFhep mice described in (A). (***, ρ ≤ 0.001). (D) WB analysis of mutant hURI-tetOFFhep mice fed with chow or NR diets. (E) Representative pictures of Sirius Red stained liver sections from hURI-tetOFFhep mice fed with chow or NR diets. n = 6. (F) Histogram depicts quantification of fibrotic area in (E). (*, ρ ≤ 0.05). (G) Representative pictures of whole livers and H&E stained liver sections from 30 week old mutant (+/Ki) hURI-tetOFFhep mice fed either chow or NR diet. Dotted yellow circles depict tumor area and black arrows depict dysplastic foci present in the mutant (+/Ki) hURI-tetOFFhep mice under chow diet. NR treatment in these mice prevents the appearance of these dysplastic foci and tumor formation. n = 5 for controls/mutants fed with chow diet and n = 9 for controls/mutants fed with NR diet. (H) WB analysis of hURI-tetOFFhep mice described in (G). (I) Representative pictures of γH2AX IHC stained liver sections from 32 week old mutant hURI-tetOFFhep mice fed with either chow or doxycycline (Dox) diets. n = 5. Scale bars represent 20 and 50 µm and 0.5 cm, as indicated.

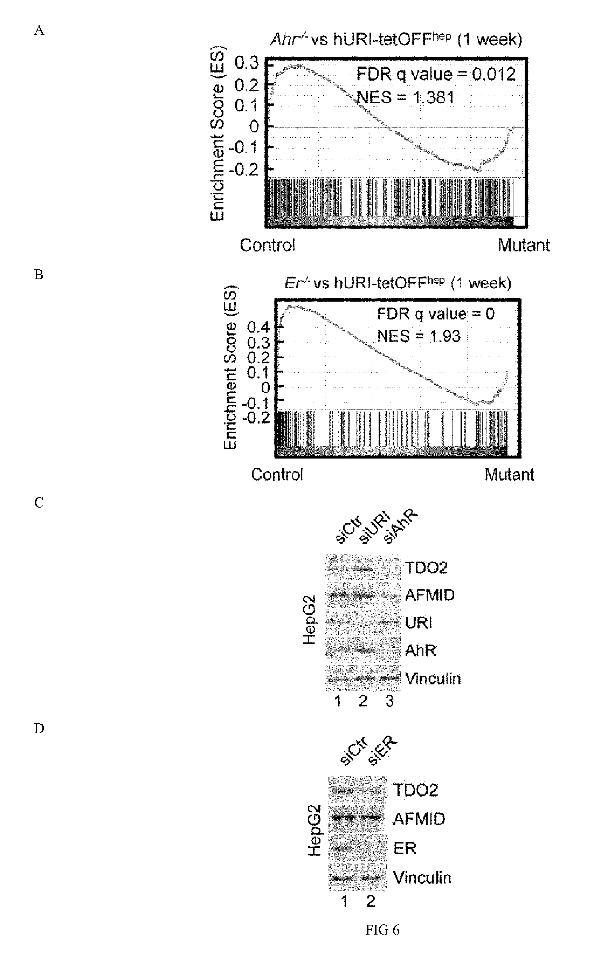

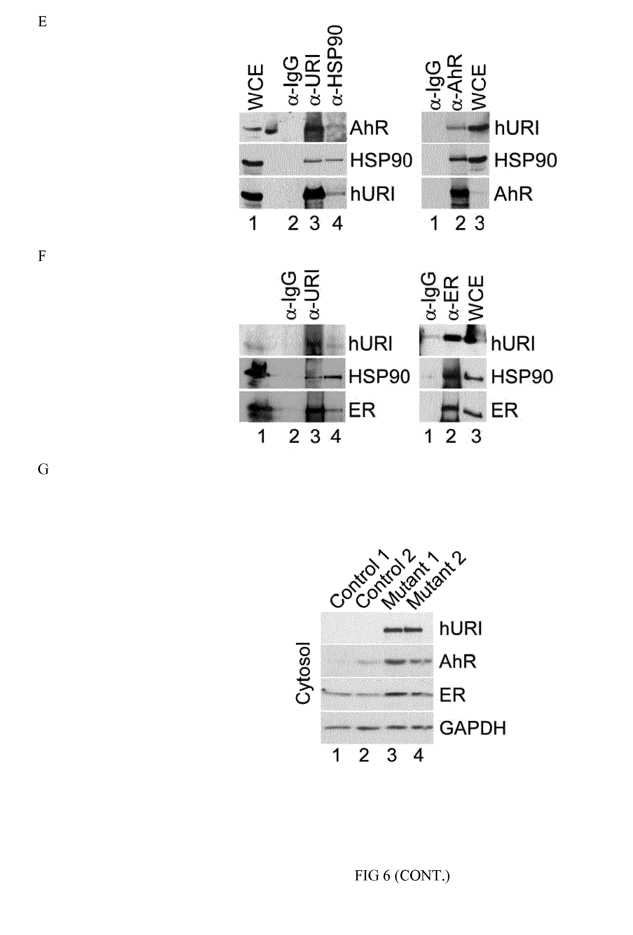

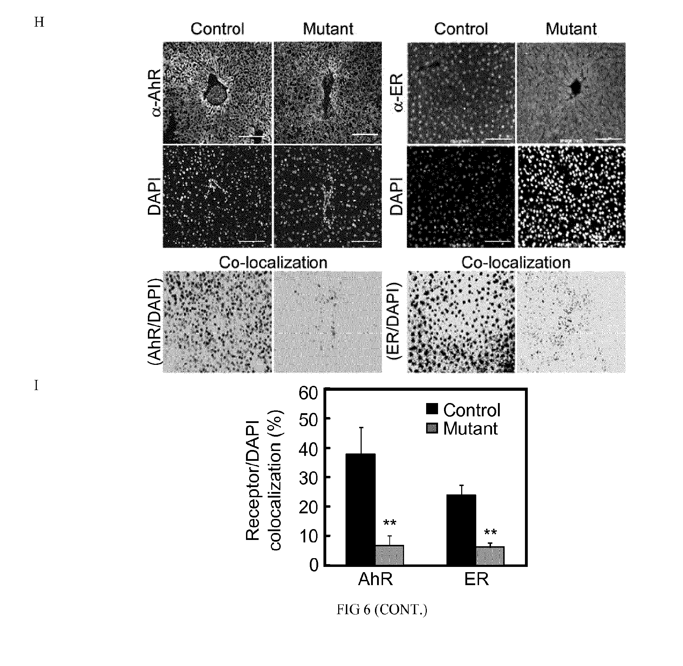

**Figure 6****. URI Regulates Kynurenine Metabolism by Modulating AhR and ER Activity.** (A and B) GSEA using livers gene arrays of *Ahr-*/*-* and *Er-*/*-* and liver RNA sequencing data from 1 week old hURI-tetOFFhep mice. (C and D) WB analysis of human HepG2 cells transfected with either scramble (siCtr) or siRNA against URI (siURI), AhR (siAhR) and ER (siER). (E and F) Immunoprecipitation using liver cytosolic fraction extracts followed by WB analysis from 1 week hURI-tetOFFhep mice with the indicated antibodies. (G) WB analysis of cytosolic fractions from 1 week old hURI-tetOFFhep mice with the indicated antibodies. (H) AhR and ER liver immunofluorescence of 1 week old control and mutant hURI-tetOFFhep mice. DAPI was used for nuclear staining. Lower panels depict nuclear co-localization done using ImageJ. n = 5 for controls and mutants. (I) Quantification of nuclear co-localization of AhR and ER shown in (h). (**, ρ ≤ 0.01). Scale bars represent 100 µm.

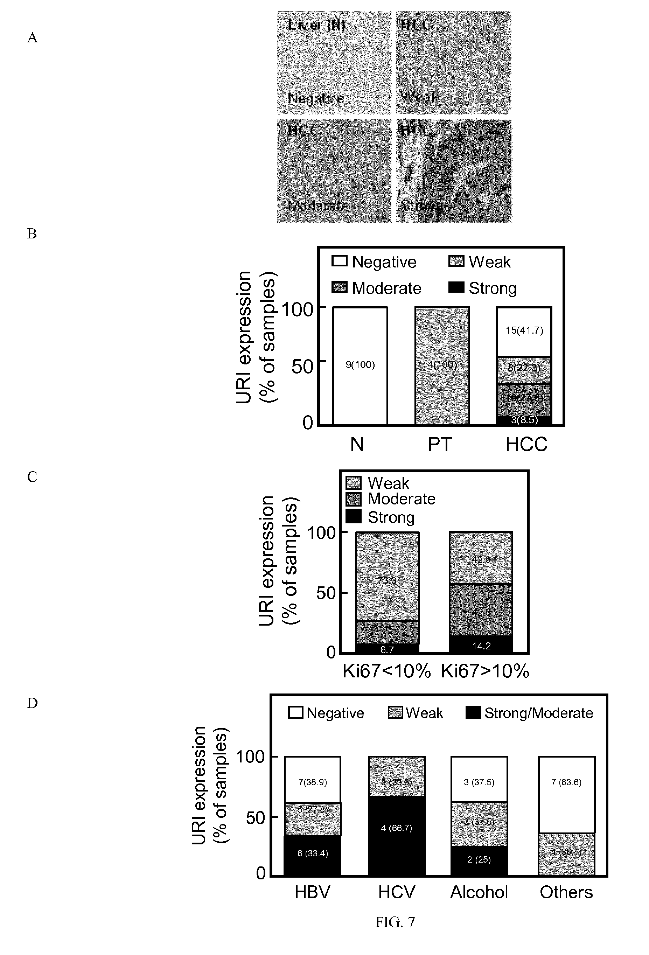

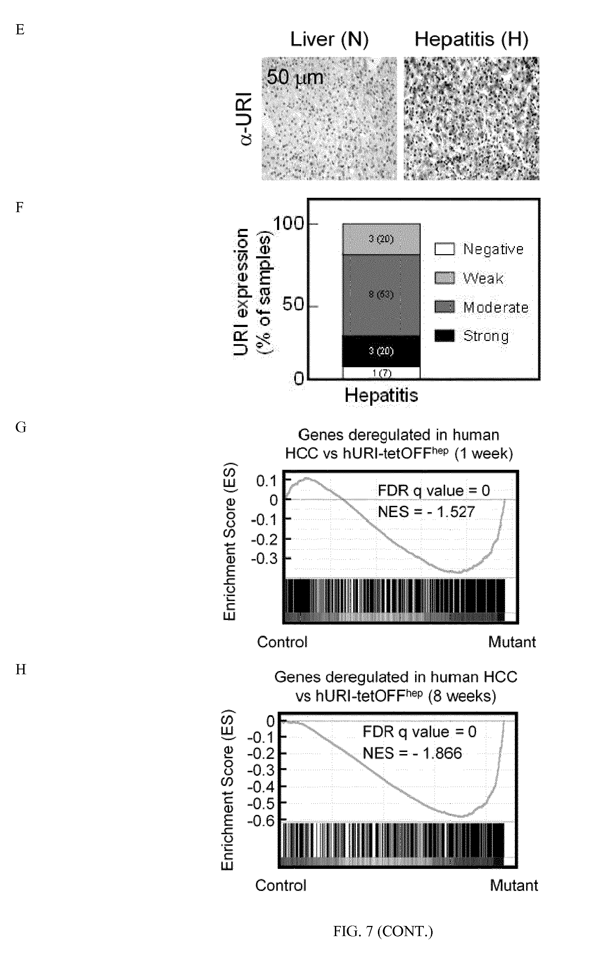

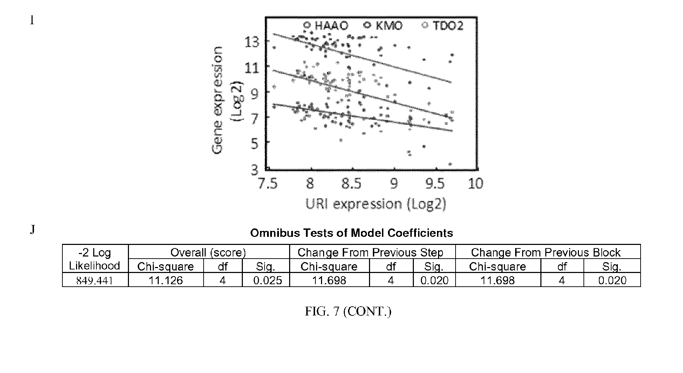

**Figure 7****. URI Expression Is Enhanced in Human HCC and Correlates with** *De* ***Novo* NAD+ Synthesis Inhibition.** (A) Representative pictures of URI IHC stained human liver sections and scoring used in the expression analysis of URI in normal liver (N) and hepatocellular carcinoma (HCC) in TMA. (B) Stratification of human samples according to the score of URI expression. (N, normal tissue; PT, peritumoral tissue and HCC tissue).Values within brackets represent percentage of total. (C) Stratification of human HCC liver samples according to the score of URI expression in less aggressive (Ki67 < 10%) and more aggressive (Ki67 > 10%) tumors. (D) Correlation of URI expression with HCC etiological factors, in HCC TMA. (E) IHC for URI in normal human liver (N) and hepatitis (H) samples. (F) Stratification of human hepatitis samples according to the score of URI expression (n = 15). Values within brackets represent percentage of total. (G and H) GSEA between differentially expressed genes in human HCC and RNA sequencing datasets from 1 (G) and 8 week (H) old hURI-tetOFFhep mice. (I) Linear regression analysis of hURI and TDO2, KMO and HAAO expressions in a human HCC microarray dataset, showing inverse correlation between hURI and TDO2, KMO, HAAO expression. (J) Multivariate Cox regression analysis for TDO2, KMO, HAAO and QPRT, in a cohort of 221 HCC patients. Overall patient survival was significantly associated with the tryptophan catabolism enzyme signature (p = 0.025).

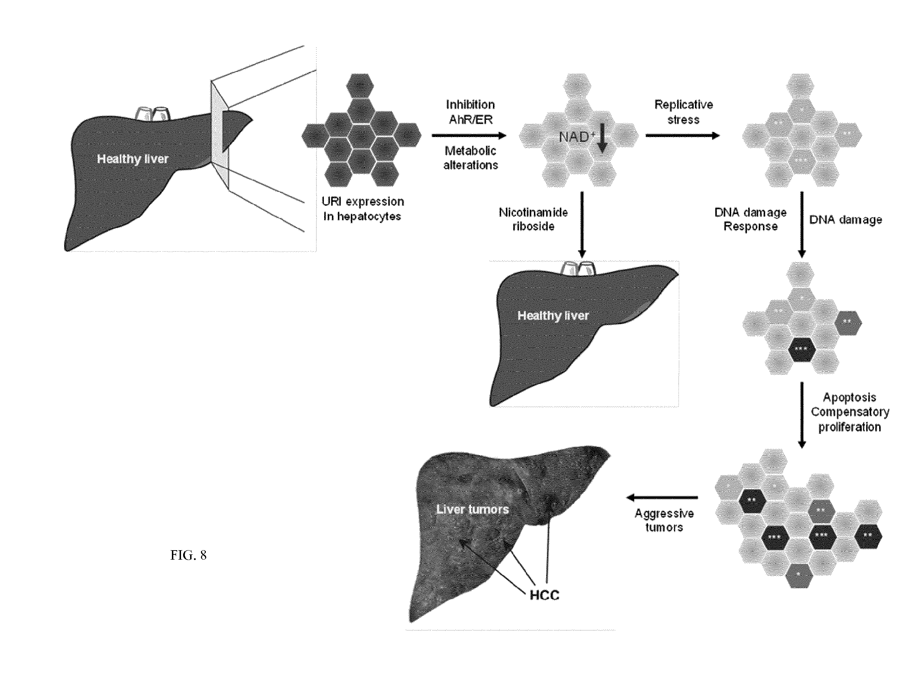

**Figure 8****. Schematic Representation of URI-Induced HCC.** Scheme representing the molecular and cellular events of hepatocarcinogenesis induced by URI expression specifically in hepatocytes.

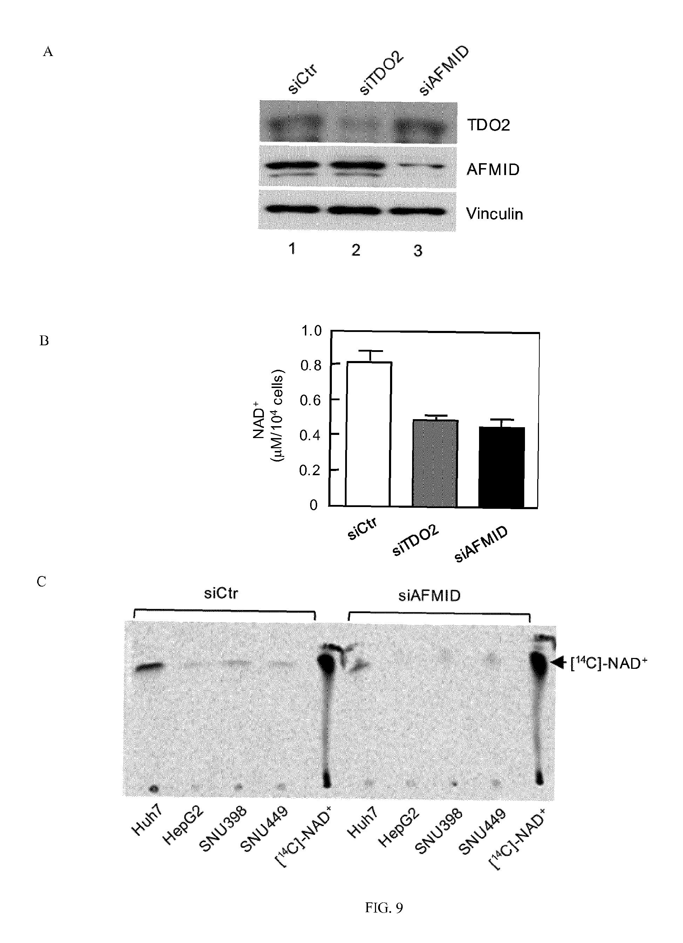

**Figure 9****. L-Tryptophan/Kynurenine Catabolism Contributes in NAD+ Synthesis.** (A) WB analysis in a HCC cell line treated with siCtr, siTDO2 or siAFMID, depicting corresponding reduction of protein levels.
   (B) Quantification of NAD+ levels in the HCC cell line described in (A). (**, ρ ≤ 0.01). (C) Representative picture of Thin Layer Chromatography (TLC) analysis of tryptophan incorporation into NAD+ via de novo NAD+ synthesis, in 4 different HCC cell lines. Silencing of Afmid (siAFMID) impairs labeled tryptophan incorporation into NAD+, thus reducing total cellular NAD+ levels. Labeled [14C]-NAD+ was used as TLC migration control.

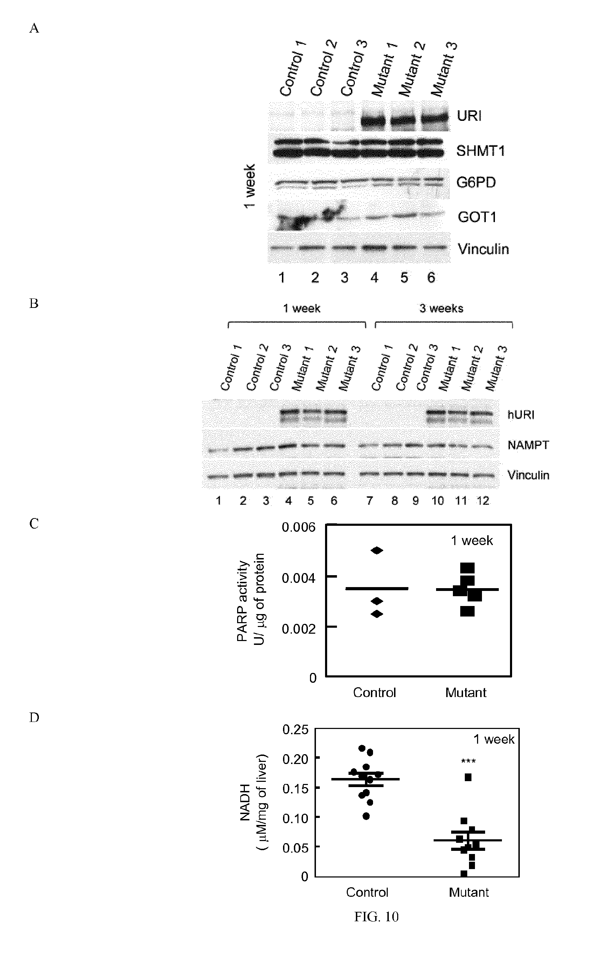

**Figure 10****. Reductions in NAD+ Levels are Not Due to Salvage Reactions and PARP Activity.** (A) WB liver analysis of important enzymes of other pathways IPA-predicted to be affected in 1 week old control and mutant hURI-tetOFFhep mice. Denote no changes in protein levels of these enzymes in the hURI expressing mutant hURI-tetOFFhep. (B) WB liver analysis of the NAMPT (NAD+ salvage pathway) enzyme in 1 and 3week old hURI-tetOFFhep mice. Denote no significant changes between control and mutant hURI- tetOFFhep mice.(C) Quantification of PARP activity in 1 week old control and mutant hURI-tetOFF hep mice. Denote no significant changes between control and mutant hURI-tetOFF hep mice. (D) Liver NADH levels in 1 week control and mutant hURI-tetOFFhep mice. Denote decreased NADH liver levels in mutant hURI-tetOFFhep mice, when compared to controls. (***, ρ ≤ 0.001).

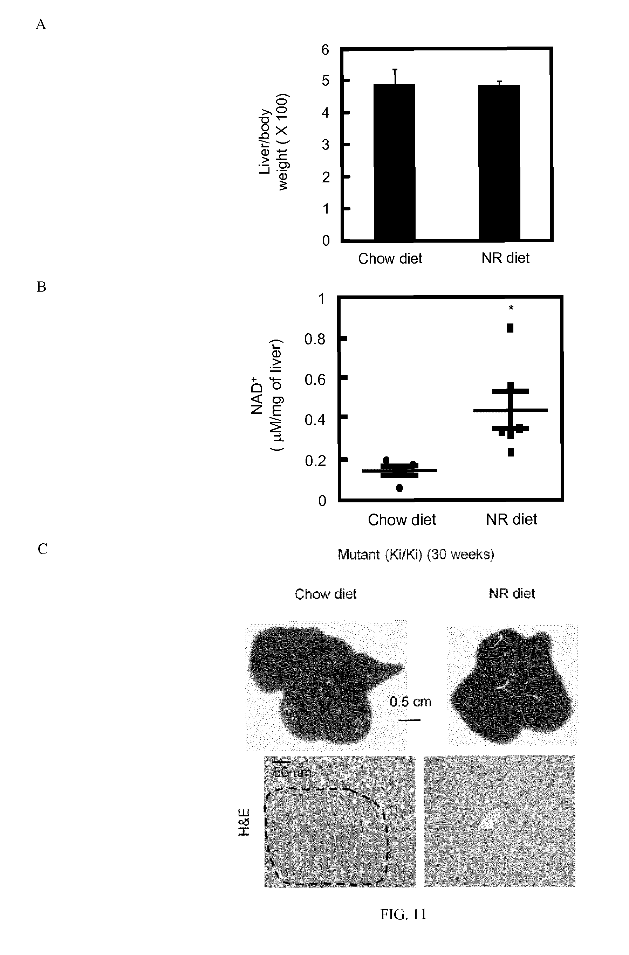

**Figure 11****. Reinstating NAD+ Pools Prevents Tumor Formation.** (A) Liver to body weight ratio in mutant hURI-tetOFFhep mice fed either chow or NR diet. (n ≥ 5). (B) Liver NAD+ levels in mutant hURI-tetOFFhep mice fed either chow or NR diet. Denote a significant increase in mutant mice feed NR diet. (*, p ≤ 0.05). (n ≥ 5). (C) Representative pictures of whole livers and H&E stained liver sections from 30 week old mutant (Ki/Ki) hURI-tetOFFhep mice fed either chow or NR diet. Dotted black circles depict tumor area and black arrows depict tumor present in the mutant (Ki/Ki) hURI-tetOFFhep mice under chow diet. NR treatment in these mice prevents the appearance tumor formation. (n ≥ 5).

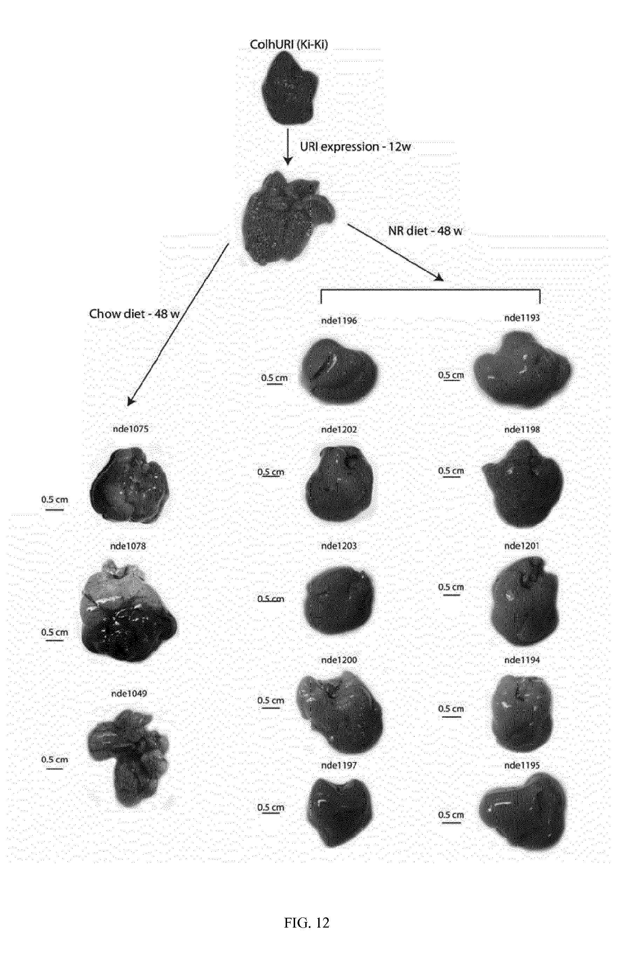

**Figure 12****. Representative pictures of whole livers from 60-week-old mutant (Ki/Ki) hURI-tetOFFhep mice fed either chow or NR diet from 12 weeks of age.**

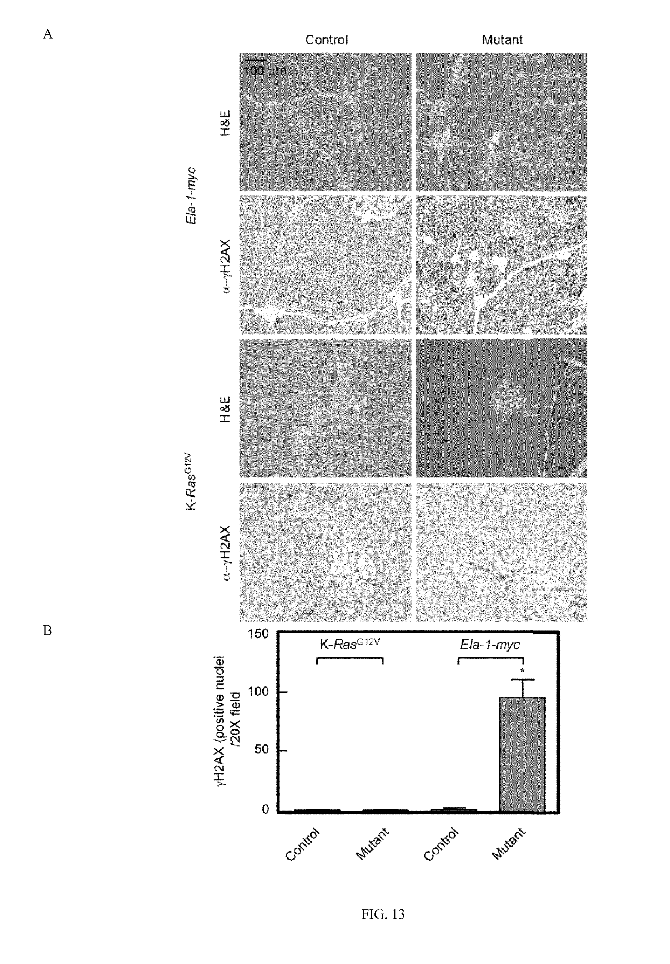

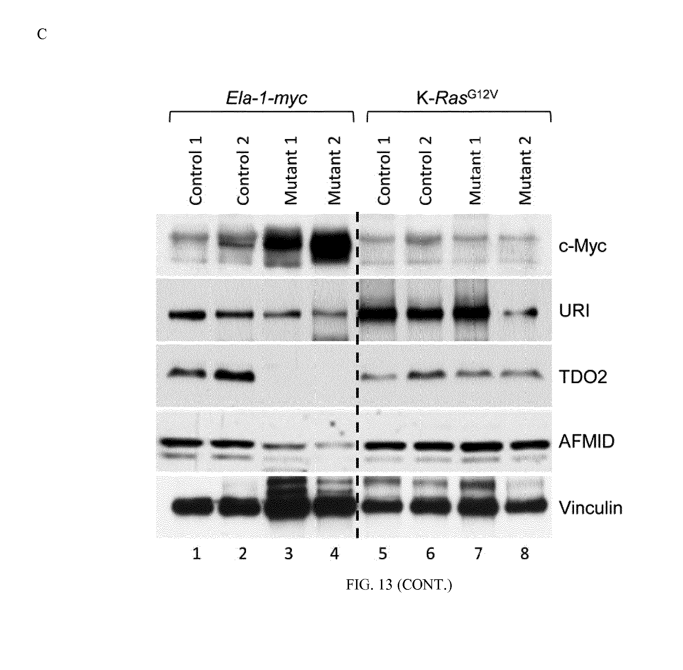

**Figure 13****. c-Myc Represses L-tryptophan/Kynurenine Catabolism and Causes DNA Damage in Early Stages of Pancreatic Adenocarcinoma Formation.** (A) Representative pictures of H&E and γH2AX stained pancreas sections from control and mutant *Ela-1-myc* and K-RAS*G12V* mice. (n ≥ 5). (B) Quantification of γH2AX positive nuclei in control and mutant *Ela-1-myc* and KRAS *G12V* mice. (*, p ≤ 0.05). (C) WB analysis of control and mutant *Ela-1-myc* and K-RAS*G12*V mice. Denote that TDO2, and AFMID protein levels decrease only in the model known to induce carcinogenesis via DNA damage pathway (Ela-1-myc) (compare lanes 3-4 to lanes 7-8). Scale bars 100µm, as indicated.

### DETAILED DESCRIPTION OF THE INVENTION

The inventors of the present invention have found that hepatocyte-specific expression of Unconventional prefoldin RPB5 Interactor (URI) leads to a multistep process of HCC development mimicking human hepatocarcinogenesis (Example 3), whereas its genetic reduction in hepatocytes protects against diethylnitrosamine (DEN)-induced HCC (Example 2). Before preneoplastic lesion formation, URI inhibits aryl hydrocarbon and estrogen receptor-mediated transcription of enzymes implicated in L-tryptophan/kynurenine catabolism leading to *de novo* nicotinamide adenine dinucleotide (NAD+) synthesis, thereby causing DNA damage (Examples 3 and 4). Restoring *in vivo* NAD+ pools with nicotinamide riboside prevents DNA damage and tumor formation (Example 5). Thus, the results demonstrate that boosting NAD+ with nicotinamide riboside can treat and prevent cancer.

### Definitions

The term "nicotinamide riboside", also known as NR, N-Ribosylnicotinamide, nicotinamide ribose, nicotinamide ribonucleoside, N-ribosylnicotinamide, 1-(β-D-Ribofuranosyl)nicotinamide, CAS number 1341-23-7, is a pyridine-nucleoside precursor to vitamin B3 and nicotinamide adenine dinucleotide (NAD+).

The term "cancer" or "tumour" or "tumour disease", as used herein, refers to a broad group of diseases involving unregulated cell growth and which are also referred to as malignant neoplasms. The term is usually applied to a disease characterized by uncontrolled cell division (or by an increase of survival or apoptosis resistance) and by the ability of said cells to invade other neighboring tissues (invasion) and spread to other areas of the body where the cells are not normally located (metastasis) through the lymphatic and blood vessels, circulate through the bloodstream, and then invade normal tissues elsewhere in the body. Depending on whether or not they can spread by invasion and metastasis, tumours are classified as being either benign or malignant: benign tumours are tumours that cannot spread by invasion or metastasis, i.e., they only grow locally; whereas malignant tumours are tumours that are capable of spreading by invasion and metastasis. Biological processes known to be related to cancer include angiogenesis, immune cell infiltration, cell migration and metastasis. Cancers usually share some of the following characteristics: sustaining proliferative signalling, evading growth suppressors, resisting cell death, enabling replicative immortality, inducing angiogenesis, and activating invasion and eventually metastasis. Cancers invade nearby parts of the body and may also spread to more distant parts of the body through the lymphatic system or bloodstream. Cancers are classified by the type of cell that the tumour cells resemble, which is therefore presumed to be the origin of the tumour.

The term "liver cancer", refers to a cancer that originates in the liver.

The term "hepatocellular carcinoma", HCC, refers to a type of liver cancer that may develop from pre-exisiting dysplastic foci or nodule. HCC arising in cirrhosis is usually preceded by the appearance of non-malignant lesions. According to gross and microscopic features HCC is differentiated into early HCC: a vaguely nodular lesion with indistinct margins with a well differentiated histology with a few portal tracts detectable within; and progressed HCC: a distinctly nodular lesion with well to moderately differentiated histology in which malignancy is recognized at first glance and no portal tracts detectable within.

"Pancreatic cancer", refers to a cancer that originates in the pancreas.

"Pancreatic ductal adenocarcinoma", PDAC, as used herein, refers to a pancreatic cancer that appears in pancreatic ductal cells.

The term "subject" or "individual" or "animal" or "patient" includes any subject, particularly a mammalian subject, for whom therapy is desired. Mammalian subjects include humans, domestic animals, farm animals, and zoo or pet animals such as dogs, cats, guinea pigs, rabbits, rats, mice, horses, cattle, cows, and so on.

"Orally", as used herein, relates to a route of administration where a substance is taken through the mouth.

"Nutritional supplementation", as used, herein refers to a product used to boost the nutritional content of the diet, that otherwise not be consumed in sufficient quantities.

"DNA damage", as used herein, relates to an alteration in the chemical structure of DNA, such as a break in a strand of DNA, a base missing from the backbone of DNA, or a chemically changed base.

"URI gene", as used herein, relates to a gene that encodes Unconventional prefoldin RPB5 interactor 1, also known as Protein NNX3, Protein phosphatase 1 regulatory subunit 19, RNA polymerase II subunit 5-mediating protein or RPB5-mediating protein. The encoded protein binds to RNA polymerase II subunit 5 (RPB5) and negatively modulates transcription through its binding to RPB5. The encoded protein seems to have inhibitory effects on various types of activated transcription, but it requires the RPB5-binding region. The sequence of URI protein in humans corresponds to the sequence 094763 in the Uniprot database 11 June 2014. Four alternatively spliced transcript variants encoding different isoforms have been described for this gene which correspond to the sequence 094763 or 094763-1, isoform 1 corresponding to the "canonical" sequence; 094763-2, isoform 2; 094763-3, isoform 3 and 094763-4, isoform 4 in the Uniprot database 11 June 2014

"c-Myc gene" refers to a regulator gene that codes for a transcription factor that activates expression of many genes through binding on consensus sequences (Enhancer Box sequences (E-boxes)) and recruiting histone acetyltransferases (HATs). The sequence of c-Myc in humans corresponds to the sequence P01106 in the Uniprot database 9 July 2014.

"Early signs of chronic liver damage", as used herein refers to a condition of the liver showing constant inflammation and/or fibrosis. Frequently the chronic liver damage is caused by hepatitis infections, particularly by hepatitis B and C. Cirrhosis is the end of chronic liver damage. Liver damage occurs on the basis on hepatocyte injury due to excessive apoptosis. Massive hepatocyte death is a feature of acute liver damage while sustained apoptosis is a feature of chronic liver damage. The term includes hepatocellular nodules, particularly hyperplastic, benign, dysplastic and malignant.

"Early signs of precancerous lesions", as used herein refers to a condition of the liver showing dysplastic foci and/or dysplastic nodules.

Dysplastic foci comprise clusters of hepatocytes, <1 mm, characterized by small (SCC) or large cell (LCC) changes.

The dysplastic nodule (adenomatous hyperplasia or AH) is a nodular lesion that differs from the surrounding liver parenchyma with regard to size, color, texture and degree of bulging of the cut surface. They are distinguished into two categories according to microscopic features:
1. low grade: a (clonal) cell population lacking architectural atypia with mild increase in cellularity as compared to surroundings and portal tracts detectable within;
2. high grade: frank cytological and architectural atypia as compared to surroundings but insufficient for a diagnosis of malignancy and portal tracts detectable within.

The term also includes the atypical adenomatous hyperplasia (AAH, AH with focally increased atypia), also called high-grade dysplatic nodule.

"Pancreatic intreaepithelial lesions", PanIN, as used herein, are microscopic papillary or flat, noninvasive epithelial neoplasms that are usually < 5 mm and confined to pancreatic ducts; composed of columnar to cuboidal cells with variable mucin, and divided into three grades according to degree of cytological and architectural atypia

The term "chemotherapeutic agent", as used herein, is an agent that at least partially inhibits the development or progression of a cancer, including inhibiting in whole or in part symptoms associated with the cancer.

"Sorafenib", also known as Nexavar, 284461-73-0, BAY 43-9006, sorafenibum, UNII-9ZOQ3TZI87, Sorafenib [INN], Kinome_766, AC1L50CF, BAY-43-9006, marketed as Nexavar by Bayer, is a small molecular inhibitor of Raf kinase, PDGF (platelet-derived growth factor), VEGF receptor 2 & 3 kinases and c Kit the receptor for Stem cell factor.

"Olaparib", 4-[(3-[(4-cyclopropylcarbonyl)piperazin-4-yl]carbonyl) -4-fluorophenyl]methyl(2H)phthalazin-1-one, as used herein, is an inhibitor of poly ADP ribose polymerase (PARP), an enzyme involved in DNA repair used as chemotherapeutic agent.

"EX-527", SEN0014196, CAS number 49843-98-3, is a SIRT1 class III histone deacetylase enzyme inhibitor.

"Hydroxyurea", or Hydroxycarbamide, CAS number 127-07-1, as used herein, refers to a compound that acts by suppressing dihydrophosphate reductase, an enzyme that reduces ribonucleotides into deoxyribonucleotides necessary for DNA synthesis.

The term "sample", as used herein refers to biological material isolated from a subject and therefore includes biological samples. Said sample can contain any biological material suitable for detecting the desired marker and can comprise cells and/or non-cellular material from the subject. In general, a sample can be isolated from any suitable biological tissue or fluid.

The term "reference value", as used herein, relates to a predetermined criteria used as a reference for evaluating the values or data obtained from the samples collected from a subject. The reference value or reference level can be an absolute value; a relative value; a value that has an upper or a lower limit; a range of values; an average value; a median value; a mean value; or a value as compared to a particular control or baseline value. A reference value can be based on an individual sample value, such as for example, a value obtained from a sample from the subject being tested, but at an earlier point in time. The reference value can be based on a large number of samples, such as from population of subjects of the chronological age matched group, or based on a pool of samples including or excluding the sample to be tested.

The term "hepatitis", as used herein, refers to a medical condition defined by the inflammation of the liver and characterized by the presence of inflammatory cells in the tissue of the organ. The condition can be self-limiting (healing on its own) or can progress to fibrosis (scarring) and cirrhosis. Viral hepatitis is the most common cause of hepatitis worldwide. Other common causes of non-viral hepatitis include toxic and drug-induced, alcoholic, autoimmune, fatty liver, and metabolic disorders. Less commonly some bacterial, parasitic, fungal, mycobacterial and protozoal infections can cause hepatitis. Additionally, certain complications of pregnancy and decreased blood flow to the liver can induce hepatitis. Cholestasis (obstruction of bile flow) due to hepatocellular dysfunction, biliary tract obstruction, or biliary atresia can result in liver damage and hepatitis.

"Hepatitis B" is an infectious illness of the liver caused by the hepatitis B virus (HBV) that affects apes, including humans. The acute illness causes liver inflammation, vomiting, jaundice, and, rarely, death. Chronic hepatitis B may eventually cause cirrhosis and liver cancer.

"Hepatitis C" is an infectious disease affecting primarily the liver, caused by the hepatitis C virus (HCV). The infection is often asymptomatic, but chronic infection can lead to scarring of the liver and

ultimately to cirrhosis, which is generally apparent after many years. In some cases, those with cirrhosis will go on to develop liver failure, liver cancer, or life-threatening esophageal and gastric varices.

The term "high probability of developing liver cancer", is understood to mean the situation where the subject shows at least 5 %, at least 10%, at least 20%, at least 30%, at least 40%, at least 50%, at least 60%, at least 70%, at least 80%, at least 90%, at least 100% probabilities of developing or suffering a liver cancer over time.

"NAD+ levels", as used herein relates to the levels of Nicotinamide adenine dinucleotide, also known as Diphosphopyridine nucleotide (DPN+) or Coenzyme I.

### 1- Therapeutic uses of the invention

The authors of the present invention have found that restoring *in vivo* NAD+ pools with nicotinamide riboside prevents DNA damage and can be used as a preventive or curative treatment in tumor formation (Example 5). Thus, the results demonstrate that boosting NAD+ with nicotinamide riboside can treat and prevent liver and pancreatic cancer.

In a first aspect, the invention relates to nicotinamide riboside or a derivative thereof for use in the treatment and/or prevention of liver cancer or pancreatic cancer in a subject.

Alternatively, the invention relates to nicotinamide riboside or a derivative thereof for use in the preparation of a medicament, nutritional composition or nutraceutical for the prevention and/or treatment of liver cancer or pancreatic cancer in a subject.

Alternatively, the invention relates to a method for treating liver cancer or pancreatic cancer in a subject in need thereof comprising administering nicotinamide riboside or a derivative therof.

In a preferred embodiment, the liver cancer is selected from hepatocellular carcinoma, adenomas or any other tumor.

In a more preferred embodiment, the liver cancer is hepatocellular carcinoma.

In another preferred embodiment, the pancreatic cancer is selected from pancreatic ductal adenocarcinoma or any other precancerous lesions or pancreatic tumors.

In a more preferred embodiment, the pancreatic cancer is pancreatic ductal adenocarcinoma

The term "prevention", "preventing" or "prevent", as used herein, relates to the administration of nicotinamide riboside or a derivative thereof according to the invention or of a medicament, nutritional composition or nutraceutical comprising said nicotinamide riboside or a derivative thereof to a subject who has not been diagnosed as possibly having a liver or pancreatic cancer at the time of administration, but who would normally be expected to develop said disease or be at increased risk for said disease. The prevention intends to avoid the appearance of liver or pancreatic cancer. The prevention may be complete (e.g. the total absence of a disease). The prevention may also be partial, such that for example the occurrence of a disease in a subject is less than that which would have occurred without the administration of the inhibitor of the present invention. Prevention also refers to reduced susceptibility to a clinical condition. This prevention is particularly useful in subjects having early signs of chronic liver damage and/or early signs of precancerous lesions in the liver, as well as those subjects suffering pancreatic intraepithelial lesions.

The term "treatment", as used herein, relates to the administration of nicotinamide riboside or a derivative thereof according to the invention or of a medicament, nutritional composition or nutraceutical comprising said nicotinamide riboside or a derivative thereof to a subject suffering from liver or pancreatic cancer including the administration in an initial or early stage of a disease, wherein the object is to prevent or slow down (lessen) an undesired physiological change or disorder. Beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. Treatment also means prolonging survival as compared to expected survival if not receiving the treatment.

"Nutritional composition" of the present invention relates to the food that beneficially affects one or more functions of the body, so as to provide better health and wellness. Accordingly, such a nutritional composition may be intended for the prevention and/or treatment of a disease or a disease causing factor. Therefore, the term "nutritional composition" of the present invention can be used as a synonym for functional food or foods for particular nutritional purposes, or medical food. A nutritional composition is similar to that of a conventional food and consumed as part of a normal diet appearance.

By "nutraceutical", a word derived from nutrition and pharmaceutical, means a product made from a food, but can be found in pill form, powders and other dosage forms not usually associated with food and having beneficial properties for the treatment and/or prevention of diseases.

The derivatives of nicotinamide riboside for use according to the invention lead to an increase of intracellular levels of nicotinamide adenine dinucleotide (NAD+).

In a preferred embodiment, the derivative of nicotinamide riboside is a compound disclosed in WO2007061798.

In another preferred embodiment the derivative of nicotinamide riboside is nicotinic acid (NA), nicotinamide (NAM), quinolinic acid or quinolinate

In a more preferred embodiment, the derivative of nicotinamide riboside is a compound having the following formula: 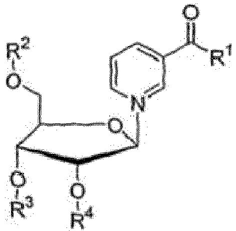 and salt complexes thereof wherein, R 1 is amine, alkylamine, substituted alkylamine, dialkylamino, substituted dialkylamino, alkyloxy, substituted alkyloxy, aryloxy, substituted aryloxy, arylthio, substituted arylthio, alkylthio, or substituted alkylthio; and R2, R3 and R4 are the same or different and, at each occurrence hydrogen, independently acyl or substituted acyl.

An illustrative, non-limitative example of a method for synthesizing derivatives of nicotinamide riboside is the method disclosed in Yang T. et al., J Med Chem. 2007 Dec 27;50(26):6458-61.

Additionally, the term derivative of nicotinamide riboside also includes pharmaceutically acceptable salts thereof.

The term "pharmaceutically acceptable salt thereof", as used herein, refers to derivatives of nicotinamide riboside wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. For example, such conventional non-toxic salts include, but are not limited to, those derived from inorganic and organic acids selected from 1,2-ethanedisulfonic, 2-acetoxybenzoic, 2-hydroxyethanesulfonic, acetic, ascorbic, benzenesulfonic, benzoic, bicarbonic, carbonic, citric, edetic, ethane disulfonic, ethane sulfonic, fumaric, glucoheptonic, gluconic, glutamic, glycolic, glycollyarsanilic, hexylresorcinic, hydrabamic, hydrobromic, hydrochloric, hydroiodide, hydroxymaleic, hydroxynaphthoic, isethionic, lactic, lactobionic, lauryl sulfonic, maleic, malic, mandelic, methanesulfonic, napsylic, nitric, oxalic, pamoic, pantothenic, phenylacetic, phosphoric, polygalacturonic, propionic, salicylic, stearic, subacetic, succinic, sulfamic, sulfanilic, sulfuric, tannic, tartaric, and toluenesulfonic.

The pharmaceutically acceptable salts of nicotinamide riboside can be synthesized from the parent compound that contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, non-aqueous media like ether, ethyl acetate, ethanol, isopropanol, or acetonitrile are useful. Lists of suitable salts are found in Remington's Pharmaceutical Sciences, 18th ed., Mack Publishing Company, Easton, Pa., 1990, p. 1445.

The derivative of nicotinamide riboside, including the pharmaceutically acceptable salt thereof for use according to the invention, promotes the increase of intracellular levels of nicotinamide adenine dinucleotide (NAD+) in cells and tissues, particularly in liver and pancreatic cells and tissues. Methods for assaying if a derivative of nicotinamide riboside, including the pharmaceutically acceptable salt thereof, is able to increase NAD+ levels are, by way of an illustrative non- limitative example, those assays based on the lactate dehydrogenase cycling reactions, in which the formed NADH reduces a formazan reagent.

The nicotinamide riboside or derivative thereof for use according to the invention may be administered by any suitable administration route, such as, but not limited to, parenteral, oral, topical, nasal, rectal route. In a particular embodiment, nicotinamide riboside or a derivative thereof for use according to the invention is administered by oral route.

Solid dosage forms for oral administration may include conventional capsules, sustained release capsules, conventional tablets, sustained-release tablets, chewable tablets, sublingual tablets, effervescent tablets, pills, suspensions, powders, granules and gels. At these solid dosage forms, the active compounds can be mixed with at least one inert excipient such as sucrose, lactose or starch. Such dosage forms can also comprise, as in normal practice, additional substances other than inert diluents, e.g. lubricating agents such as magnesium stearate. In the case of capsules, tablets, effervescent tablets and pills, the dosage forms may also comprise buffering agents. Tablets and pills can be prepared with enteric coatings. Enteric coatings may be applied using conventional processes known to experts in the art, as described in, for example, Johnson, J. L., "Pharmaceutical tablet coating", Coatings Technology Handbook (Second Edition), Satas, D. and Tracton, A. A. (eds), Marcel Dekker, Inc. New York, (2001), Carstensen, T., "Coating Tablets in Advanced Pharmaceutical Solids", Swarbrick, J. (ed.), Marcel Dekker, Inc. New York (2001), 455-468.

In one embodiment of the invention, the orally administrable form of nicotinamide riboside or derivative thereof of the invention is in a sustained release form further comprising at least one coating or matrix. The coating or sustained release matrix include, without limitation, natural polymers, semisynthetic or synthetic water-insoluble, modified, waxes, fats, fatty alcohols, fatty acids, natural semisynthetic or synthetic plasticizers, or a combination of two or more of the them.

Even though individual needs vary, determination of optimal ranges for effective amounts of nicotinamide riboside or derivative thereof of the invention belongs to the common experience of those experts in the art. In general, the dosage needed to provide an effective treatment, which can be adjusted by one expert in the art, will vary depending on age, health, fitness, sex, diet, weight, degree of alteration of URI levels, frequency of treatment and the nature and extent of impairment or illness, medical condition of the patient, route of administration, pharmacological considerations such as activity, efficacy, pharmacokinetic and toxicology profile of the particular compound used, if using a system drug delivery, and if the compound is administered as part of a combination of drugs.

In a preferred embodiment, the nicotinamide riboside or a derivative thereof for use according to the invention is administered from 100 mg to 5 g a day, more preferably from 500 mg to 2 g a day. The description of a range should be considered to have specifically disclosed all the possible subranges as well as individual numerical values within that range.

In another preferred embodiment, the nicotinamide riboside or a derivative thereof is administered as a nutritional supplementation. In a preferred embodiment, nicotinamide riboside or a derivative thereof for use according to the invention is administered as a nutritional composition or nutraceutical.

Nutritional supplement comprising nicotinamide riboside or a derivative thereof can be made in a variety of forms such as a pharmaceutical composition (e.g., table, powder, suspension, liquid, capsule, and gel), nutritional beverages, puddings, confections (i.e., candy), ice cream, frozen confections, or non-baked extruded food. The nutritional supplement can be formulated into a snack to be taken as part of a diet.

In another preferred embodiment, the subject is a human. In another preferred embodiment, the subject to be treated with the nicotinamide riboside or a derivative thereof shows high level of expression of a gene inducing DNA damage.

The skilled person knows genes that induce DNA damage. Illustrative, non-limitative examples of genes that induce DNA damage are c-Myc, URI, AP-1 transcription factors (including cFOS, cJUN, JUNB, FRA-1, FRA-2 and ATF), oncogenes from the DNA damage response.

Additionally, the skilled person knows several methods for determining if a particular gene induces DNA damage. By way of non-limitative example, PCR (polymerase chain reaction), comet, halo, TUNEL (Terminal deoxyribonucleotidyltransferase-mediated deoxyuridine triphosphate nick end labeling) assay, HPLC-Electrospray tandem mass spectrometry, FISH (Fluorescence in situ hybridization), FCM (Flow cytometry), annexin V labeling, immunological assays including immunofluorescent and chemiluminescence thymine dimer detection, immunohistochemical assay, Enzyme-linked immunosorbent assay (ELISA), Radio immunoassay (RIA), Gas chromatography-mass spectrometry and electrochemical methods.

In a preferred embodiment, the subject to be treated according to the invention for the prevention and/or treatment of liver cancer shows high level of expression of URI gene.

In another preferred embodiment, the subject to be treated according to the invention for the prevention and/or treatment of pancreatic cancer shows high level of expression of c-Myc gene.

URI or c-Myc levels are considered to be higher with respect to a corresponding reference value when the levels of URI or c-Myc in a sample show an increase of at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150% or more. The reference value can be the value corresponding to the level of expression of URI or c-Myc in a non-cancer sample.

As a person skilled in the art can know, the expression of a gene can also be detected by measuring the expression level of a functionally equivalent variant of URI or c-Myc and different isoforms of URI. In a preferred embodiment, the isoform of URI to be detected is RMP (RBP5-mediating protein).

"Functionally equivalent variant" is understood to mean all those proteins derived from URI sequence or c-Myc sequence by modification, insertion and/or deletion of one or more amino acids, whenever the function is substantially maintained.

Assays to determine the function of an enzyme are known by the skilled person and include, without limitation, initial rate assays, progress curve assays, transient kinetics assays and relaxation assays. Continuous assays of enzymatic activity include, without limitation, spectrophotometric, fluorometric, calorimetric, chemiluminiscent, light scattering and microscale thermopheresis assays. Discontinuous assays of enzymatic activity include, without limitation, radiometric and chromatographic assays. As the skilled person understands, factors that may influence enzymatic activity comprise salt concentration, temperature, pH, and substrate concentration

The activity of URI can be determined by detecting the downregulation of any enzyme implicated in the L-tryptophan/kynureine degradation, including tryptophan 2,3-dioxygenase (TDO2) and arylformamidase (AFMID) catalyzing the initial rate-limiting step of tryptophan degradation, kynurenine 3-monooxygenase (KMO), kynureninase (KNYU) and 3-hydroxyanthranilate 3,4-dioxygenase (HAAO). Additionally, the activity of URI can be determined by detecting a decrease of NAD+ levels in the cell. Assays to determine the NAD+ levels have been previously described.

Alternatively, URI activity can be monitored via activity of *O*-linked N-acetylglucosamine (*O-GlcNAc)* transferase (*OGT*), activity of S6K1 or any other kinases that can be activated through Threonine and Serine phosphorylation and regulated by PP1 phosphatase. URI activity can also be monitored by measurement of PP1 activity, folding of proteins and RNA polymerase I, II and III activity (pausing and transcriptional activity).

The activity of c-Myc can be determined by analyzing its transcriptional activity, by way of non-limitative example, by measuring the repression of mRNA expression of the platelet-derived growth factor receptor beta gene (PDGFRB). Any reporter assays to monitor Myc activity would be useful.

Preferably, variants of URI or c-Myc are (i) polypeptides in which one or more amino acid residues are substituted by a preserved or non-preserved amino acid residue (preferably a preserved amino acid residue) and such substituted amino acid may be coded or not by the genetic code, (ii) polypeptides in which there is one or more modified amino acid residues, for example, residues modified by substituent bonding, (iii) polypeptides resulting from alternative processing of a similar mRNA, (iv) polypeptide fragments and/or (v) polypeptides resulting from URI fusion or the polypeptide defined in (i) to (iii) with another polypeptide, such as a secretory leader sequence or a sequence being used for purification (for example, His tag) or for detection (for example, Sv5 epitope tag). The fragments include polypeptides generated through proteolytic cut (including multisite proteolysis) of an original sequence. The variants may be post-translationally or chemically modified. Such variants are supposed to be apparent to those skilled in the art.

As known in the art, the "similarity" between two polypeptides is determined by comparing the amino acid sequence and the substituted amino acids preserved from a polypeptide with the sequence of a second polypeptide. The variants are defined to include polypeptide sequences different from the original sequence, preferably different from the original sequence in less than 40% of residues per segment concerned, more preferably different from the original sequence in less than 25% of residues per segment concerned, more preferably different from the original sequence in less than 10% of residues per segment concerned, more preferably different from the original sequence in only a few residues per segment concerned and, at the same time, sufficiently homologous to the original sequence to preserve functionality of the original sequence. The present invention includes amino acid sequences which are at least 60%, 65%, 70%, 72%, 74%, 76%, 78%, 80%, 90%, or 95% similar or identical to the original amino acid sequence. The degree of identity between two polypeptides may be determined using computer algorithms and methods which are widely known to those skilled in the art. The identity between two amino acid sequences is preferentially determined using BLASTP algorithm [BLASTManual, Altschul, S. et al., NCBI NLM NIH Bethesda, Md. 20894, Altschul, S., et al., J. Mol. Biol. 215: 403-410 (1990)].

Methods for detecting the expression of a gene can be based on detecting mRNA or protein, or they also can be based on determining the mRNA levels or protein levels and the levels of variants thereof, in a sample as a whole, in cells of a sample and/or in the non-cellular fraction of a sample.

Methods for detecting mRNA are well known in the art and include without limitation, standard assays for determining mRNA expression levels such as qPCR, RT-PCR, RNA protection analysis, Northern blot, RNA dot blot, *in situ* hybridization, microarray technology, tag based methods such as serial analysis of gene expression (SAGE) including variants such as LongSAGE and SuperSAGE, microarrays, fluorescence *in situ* hybridization (FISH), including variants such as Flow-FISH, qFiSH and double fusion FISH (D-FISH), and the like. Preferably quantitative or semi-quantitative RT-PCR is preferred. Real-time quantitative or semi-quantitative RT-PCR is particularly advantageous.

In case the mRNA is measured in a biological sample, said biological sample may be treated to physically, mechanically or chemically disrupt tissue or cell structure, to release intracellular components into an aqueous or organic solution to prepare nucleic acids for further analysis. The nucleic acids are extracted from the sample sample (e.g., cell or tissue prepared from the subject) by procedures known to the skilled person and commercially available, for example using lytic enzymes or chemical solutions or extracted by nucleic-acid-binding resins following the manufacturer's instructions. RNA is then extracted from frozen or fresh samples by any of the methods typical in the art, for example, Sambrook, J., et al., 2001. Molecular cloning: A Laboratory Manual, 3rd ed., Cold Spring Harbor Laboratory Press, N.Y., Vol. 1-3. Preferably, care is taken to avoid degradation of the RNA during the extraction process.

The expression level can be determined using mRNA obtained from a formalin-fixed, paraffin-embedded tissue sample. mRNA may be isolated from an archival pathological sample or biopsy sample which is first deparaffinized. An exemplary deparaffinization method involves washing the paraffinized sample with an organic solvent, such as xylene. Deparaffinized samples can be rehydrated with an aqueous solution of a lower alcohol. Suitable lower alcohols, for example, include methanol, ethanol, propanols and butanols. Deparaffinized samples may be rehydrated with successive washes with lower alcoholic solutions of decreasing concentration, for example. Alternatively, the sample is simultaneously deparaffinized and rehydrated. The sample is then lysed and RNA is extracted from the sample. Samples can be also obtained from fresh tumor tissue such as a resected tumor. In a particular embodiment samples can be obtained from fresh tumor tissue or from OCT embedded frozen tissue.

In order to normalize the values of mRNA expression among the different samples, it is possible to compare the expression levels of the mRNA of interest in the test samples with the expression of a control RNA. A "control RNA", as used herein, relates to RNA whose expression levels do not change or change only in limited amounts. Preferably, the control RNA is mRNA derived from housekeeping genes and which code for proteins which are constitutively expressed and carry out essential cellular functions. Preferred housekeeping genes for use in the present invention include 18-S ribosomal protein, β-2-microglobulin, ubiquitin, cyclophilin, GAPDH, PSMB4, tubulin and β-actin.

The relative gene expression quantification may be calculated according to the comparative threshold cycle (Ct) method using a housekeeping gene as an endogenous control and commercial RNA controls as calibrators. Final results are determined according to the formula 2-(ΔCt sample-ΔCt calibrator), where ΔCt values of the calibrator and sample are determined by subtracting the Ct value of the target gene from the value of the control gene.

Alternatively, it is also possible to determine the expression level of a gene by means of the determination of the expression levels of the proteins encoded by said gene, since if the expression of the gene is increased, an increase of the amount of corresponding proteins should occur and if the expression of the gene is decreased, a decrease of the amount of corresponding proteins should occur.

Virtually any conventional method can be used within the frame of the invention to detect and quantify the levels of proteins. By way of a non-limiting illustration, the expression levels are determined by means of antibodies with the capacity for binding specifically to the protein to be determined (or to fragments thereof containing the antigenic determinants) and subsequent quantification of the resulting antigen-antibody complexes. The antibodies that are going to be used in this type of assay can be, for example, polyclonal sera, hybridoma supernatants or monoclonal antibodies, antibody fragments, Fv, Fab, Fab' and F(ab')2, scFv, diabodies, triabodies, tetrabodies and humanized antibodies. At the same time, the antibodies may or may not be labeled. Illustrative, but non-exclusive, examples of markers that can be used include radioactive isotopes, enzymes, fluorophores, chemoluminescent reagents, enzyme cofactors or substrates, enzyme inhibitors, particles, dyes, etc. There is a wide variety of well-known assays that can be used in the present invention, using non-labeled antibodies (primary antibody), labeled antibodies (secondary antibodies); these techniques include Western-blot or immunoblot, ELISA (enzyme-linked immunosorbent assay), RIA (radioimmunoassay), competitive EIA (enzyme immunoassay), DAS-ELISA (double antibody sandwich ELISA), immunocytochemical and immunohistochemical techniques, immunofluorescence, techniques based on the use of biochips or protein microarrays including specific antibodies or assays based on the colloidal precipitation in formats such as reagent strips. Other forms of detecting and quantifying the proteins include affinity chromatography techniques, ligand-binding assays, etc.

On the other hand, the determination of the levels of a protein can be carried out by constructing a tissue microarray (TMA) containing the subject samples assembled, and determining the expression levels of the corresponding protein by immunohistochemistry techniques. Immunostaining intensity can be evaluated by two or more different pathologists and scored using uniform and clear cut-off criteria, in order to maintain the reproducibility of the method. Discrepancies can be resolved by simultaneous re-evaluation. Briefly, the result of immunostaining can be recorded as negative expression (0) versus positive expression, and low expression (1+) versus moderate (2+) and high (3+) expression, taking into account the expression in tumor cells and the specific cut-off for each marker. As a general criterion, the cut-offs are selected in order to facilitate reproducibility, and when possible, to translate biological events. Alternatively, the immunostaining intensity can be evaluated by using imaging techniques and automated methods such as those disclosed in Rojo, M.G. et al. (Folia Histochem. Cytobiol. 2009; 47: 349-54) or Mulrane, L. et al. (Expert Rev. Mol. Diagn. 2008; 8: 707-25).

In another preferred embodiment, the subject to be treating using nicotinamide riboside or a derivative thereof shows early signs of chronic liver damage or pancreatic intraepithelial lesions. In a more preferred embodiment, the early signs of chronic liver damage or pancreatic intraepithelial lesions are early signs of precancerous lesions.

In another preferred embodiment, nicotinamide riboside or a derivative thereof, for use according to the invention, is administered in combination with a chemotherapeutic agent.

Illustrative, non- limitative examples of chemotherapeutics agents are shown in Table 1.

| TABLE 1. CHEMOTHERAPEUTICS AGENTS SUITABLE FOR USE ACCORDING TO THE INVENTION | |
|---|---|
| Enzyme inhibitors | - Tyrosine kinase inhibitors such as Sorafenib, Genistein (4', 5, 7-trihydroxyisoflavone), Tyrphostin 25 (3,4,5-trihydroxyphenyl), methylene]- propanedinitrile, Herbimycin A, Daidzein (4',7-dihydroxyisoflavone), AG-126, trans-1- (3'-carboxy-4'-hydroxyphenyl)-2-(2",5"-dihydroxy-phenyl)ethane |
| | - MAP kinase inhibitors such as KY12420 (C23H24O8), CNI-1493, PD98059, or 4-(4- Fluorophenyl)-2-(4-methylsulfinyl phenyl)-5-(4-pyridyl) 1H-imidazole |
| | - EGFR inhibitors such as erlotinib (TARCEVA), gefitinib (IRESSA), WHI- P97 (quinazoline derivative), LFM-A12 (leflunomide metabolite analog), ABX-EGF, lapatinib, canertinib, ZD-6474 (ZACTIMA), AEE788, and AG1458 |
| | - VEGF inhibitors such as bevacizumab (AVASTIN), ranibizumab (LUCENTIS), pegaptanib (MACUGEN), sorafenib, sunitinib (SUTENT), vatalanib, ZD-6474 (ZACTIMA), anecortave (RETAANE), squalamine lactate, and semaphorin, trastuzumab (HERCEPTIN), alemtuzumab (CAMPATH), gemtuzumab (MYLOTARG, hP67.6, anti-CD33,), rituximab (RITUXAN), tositumomab (BEXXAR, anti-CD20), MDX-210, oregovomab (OVAREX), edrecolomab (PANOREX), daclizumab (ZENAPAX), palivizumab (SYNAGIS), ibritumomab tiuxetan (ZEVALIN, cetuximab (ERBITUX), MDX-447, MDX-22, MDX-220 (anti-TAG-72), I0R-C5, I0R-T6 (anti-CD 1), IOR EGF/R3, celogovab (ONCOSCINT OV 103), epratuzumab (LYMPHOCIDE), pemtumomab (THERAGYN), and Gliomab-H |
| | - NAD-dependent deacetylase inhibitors, such as EX-572 |
| DNA damaging agents | Olaparib, etoposide, ramptothecin, topotecan, teniposide, mitoxantrone |
| DNA alkylating agents | cisplatin, mechlorethamine, cyclophosphamide, ifosfamide, melphalan, chorambucil, busulfan, thiotepa, carmustine, lomustine, carboplatin, dacarbazine, procarbazine |
| DNA strand break inducing agents | bleomycin, doxorubicin, daunorubicin, idarubicin, mitomycin |
| Anti-microtubule agents | vincristine, vinblastine |
| Anti-metabolic agents | cytarabine, methotrexate, hydroxyurea, 5-fluorouracil, floxuridine, 6-thioguanine, 6-mercaptopurine, fludarabine, pentostatin, chlorodeoxyadenosine |

In a preferred embodiment, the chemotherapeutic agent is a tyrosine kinase inhibitor. In a more preferred embodiment, the chemotherapeutic agent is sorafenib.

In another preferred embodiment, the chemotherapeutic agent is selected from the group consisting of sorafenib, olaparib, hydroxyurea, EX-572 and combinations thereof. Thus, the invention also contemplates the combination of nicotinamide riboside or a derivative thereof with two or more chemotherapeutic agents.

### 2- In vitro methods for designing a customized therapy

In a second aspect, the invention relates to an *in vitro* method for designing a customized therapy for a subject diagnosed with a liver cancer or pancreatic cancer, suffering early signs of chronic liver damage or suffering pancreatic intraepithelial lesions which comprises
a) determining the level of DNA damage in a sample from said subject, and
b) comparing said level with a reference value,
wherein an increased level of DNA damage in said sample with respect to the reference value is indicative that the subject is to be treated with nicotinamide riboside or a derivative thereof.

The DNA damage can be induced by pathogens such as bacteria or viruses or by expression of a gene that induces DNA damage and can be determined using any method known in the art. By way of illustrative, non-limitative example measuring the level of expression of a gene inducing DNA damage, detecting chemical modifications in the DNA such as SSB (single strand break), DSB (double strand break), CPDs (cyclobutane pyrimidine dimers), 6-4PPs (6-4 photoproducts) by methods such as PCR (polymerase chain reaction), comet, halo, TUNEL (Terminal deoxyribonucleotidyltransferase-mediated deoxyuridine triphosphate nick end labeling) assay, HPLC-Electrospray tandem mass spectrometry, FISH (Fluorescence in situ hybridization), FCM (Flow cytometry), annexin V labeling, immunological assays including immunofluorescent and chemiluminescence thymine dimer detection, immunohistochemical assay, Enzyme-linked immunosorbent assay (ELISA), Radio immunoassay (RIA), Gas chromatography-mass spectrometry and electrochemical methods.

In a preferred embodiment, the level of DNA damage is considered to be higher with respect to a corresponding reference value when the level of DNA damage in a sample shows an increase of at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150% or more.

In a preferred embodiment the DNA damage is detected by determining the level of expression of a gene inducing DNA damage. In a preferred embodiment, the gene inducing DNA damage is selected from c-Myc, URI, AP-1 transcription factors including cFOS, cJUN, JUNB, FRA-1, FRA-2 and ATF. In a more preferred embodiment the gene inducing DNA damage is selected from URI and c-Myc.

In a preferred embodiment, the method for designing a customized therapy for a subject diagnosed with a liver cancer or suffering early signs of chronic liver damage comprises determining the level of expression of URI gene.

In another preferred embodiment, the method for designing a customized therapy for a subject diagnosed with pancreatic cancer or suffering pancreatic intraepithelial lesions comprises determining the level of expression of c-Myc.

URI or c-Myc levels are considered to be higher with respect to a corresponding reference value when the levels of URI or c-Myc in a sample show an increase of at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150% or more. The reference value can be the value corresponding to the level of expression of URI or c-Myc in a non-cancer sample.

In another preferred embodiment, the method for designing a customized therapy further comprises determining the glucose uptake versus glutamine uptake. In another preferred embodiment, the DNA damage is determined by detecting DNA damage markers, such as 8-hydroxy-2-deoxguanosine (8-OHdG) in blood and liver biopsies and/or γH2Ax, P-ATM, P-ATR, P-CHK1/2 and P-RPA in a sample.

In a preferred embodiment, the sample is blood, liver or pancreatic biopsies.

In a preferred embodiment of the method for designing a customized therapy, the liver cancer is hepatocellular carcinoma

In another preferred embodiment, the early signs of chronic liver damage are caused by hepatitis.

In a preferred embodiment of the second method of the invention, the pancreatic cancer is pancreatic ductal adenocarcinoma.

The term "therapy", as used herein, refers to the attempted remediation of a health problem, usually following a diagnosis, or to prevention or the appearance of a health problem. As such, it is not necessarily a cure, i.e. a complete reversion of a disease. Said therapy may or may not be known to have a positive effect on a disease, such as liver cancer. This term includes both therapeutic treatment and prophylactic or preventative measures, in which the object is to prevent or stop (reduce) an undesired physiological change or disorder. For the purpose of this invention, beneficial or desired clinical results include, without limitation, relieving symptoms, reducing the spread of the disease, stabilizing pathological state (specifically not worsening), slowing down or stopping the progression of the disease, improving or mitigating the pathological state and remission (both partial and complete), both detectable and undetectable. It can also involve prolonging survival in comparison with the expected survival if treatment is not received. Those subjects needing treatment include those subjects already suffering the condition or disorder, as well as those with the tendency to suffer the condition or disorder or those in which the condition or disorder must be prevented.

In a preferred embodiment, the subject is a human.

In a first step, the first method of the invention comprises determining the level of expression of URI gene in a sample from the subject and the second method of the invention comprises determining the level of expression of c-Myc gene in a sample from the subject.

The expression "determining the expression level", as used herein, refers to determining the level of expression of a biomarker. Therein, the level of expression refers to the level of mRNA and/or the level of protein. Assays to analyzing the levels of expression of URI gene have been previously described. In a preferred embodiment of the invention, detecting the expression or determining the levels of a gene is performed by Western blot or PCR.

In a preferred embodiment of the first method of the invention, the sample is a sample from hepatic precancerous lesions or the liver tumor including all tumor types. In a preferred embodiment of the second method of the invention, the sample is a sample from precancerous lesions or pancreatic tumor including all tumor types.

The second step of the methods of the invention comprises comparing the levels of expression of the gene in a sample from the subject with a reference value. Thus, if a sample of the subject shows an increased level of expression of URI or c-Myc gene with respect to the corresponding reference value, is indicative that the subject is to be treated with nicotinamide riboside or a derivative thereof. In a preferred embodiment nicotinamide riboside or a derivative thereof is administered orally, more preferred as a nutritional supplementation, nutraceutical or nutritional composition.

All the terms and embodiments previously described are equally applicable to this aspect of the invention.

### 3- Diagnostic method of the invention

In another aspect, the invention relates to an *in vitro* method for diagnosing liver cancer in a subject which comprises:
a) determining the level of expression of URI gene in a sample from said subject, and
b) comparing said level with a reference value,
wherein an increased level of expression of URI gene in said sample with respect to the reference value is indicative that the subject suffers liver cancer.

Diagnosing, as used herein, refers both to the process of attempting to determine and/or identify a possible disease in a subject, i.e. the diagnostic procedure, and to the opinion reached by this process, i.e. the diagnostic opinion. As such, it can also be regarded as an attempt at classification of an individual's condition into separate and distinct categories that allow medical decisions about treatment and prognosis to be made. As will be understood by those skilled in the art, the diagnosis of liver cancer, although preferred to be, need not be correct for 100% of the subjects to be diagnosed or evaluated. The term, however, requires that a statistically significant portion of subjects can be identified as suffering liver cancer. Whether a subject is statistically significant can be determined without further ado by the person skilled in the art using various well known statistic evaluation tools, e.g., determination of confidence intervals, p-value determination, Student's t-test, Mann-Whitney test, etc. Details are found in Dowdy and Wearden, Statistics for Research, John Wiley & Sons, New York 1983. Preferred confidence intervals are at least 50%, at least 60%, at least 70%, at least 80%, at least 90%, or at least 95%. The p-values are, preferably, 0.05, 0.01, 0.005 or lower.

The first and second step of the diagnostic method of the invention can be performed as previously described in relation to the first and second method of the invention.

The reference value may be derived from a sample collection formed preferably by a mixture of the sample to be analyzed from normal individuals not affected by a cancer. Said reference value can be determined by means of techniques well known in the state of the art, for example, determining the mean of the levels of URI gene measured in a sample taken from healthy subjects. The reference value can also be obtained from the constitutively expressed proteins taken from the same subject to be analyzed.

All the terms and embodiments previously described are equally applicable to this aspect of the invention.

### 4- Method for predicting the risk of developing liver cancer

In another aspect, the invention relates to an *in vitro* method for predicting the risk of developing liver cancer in a subject suffering hepatitis which comprises:
a) determining the level of expression of URI gene in a sample from said subject, and
b) comparing said levels with a reference value,
wherein an increased level of expression of URI gene in said sample with respect to the reference value is indicative that the subject shows high risk of developing liver cancer.

As used herein, the term "prediction method" refers to a method for determining the probability that a patient suffers liver cancer, particularly hepatocellular carcinoma. The person skilled in the art will estimate that the prediction may not be correct for 100% of patients under study. However, the expression requires that the prediction method provides correct results for a statistically significant portion of patients. Determination whether the method of the invention provides statistically significant predictions can be carried out by using standard statistical techniques such as the determination of confidence intervals, determination of p value, Student's t-test, Mann-Whitney test as described in Dowdy and Wearden, Statistics for Research, John Wiley & Sons, New York 1983. Suitable confidence intervals are at least 50%, at least 60%, at least 70%, at least 80%, at least 90%, at least 95%. p values are, preferably, 0.2, 0.1, 0.05.

The first and second step of the predicting method of the invention can be performed as previously described in relation to the first and second method of the invention.

All the terms and embodiments previously described are equally applicable to this aspect of the invention.

### 5-Kit of the invention

In another aspect, the invention relates to a kit comprising a reagent which allows determining the expression level of URI gene.

In the context of the present invention, "kit" is understood as a product containing the different reagents necessary for carrying out the methods of the invention packed so as to allow their transport and storage. Additionally, the kits of the invention can contain instructions for the simultaneous, sequential or separate use of the different components which are in the kit. Said instructions can be in the form of printed material or in the form of an electronic support capable of storing instructions susceptible of being read or understood, such as, for example, electronic storage media (e.g. magnetic disks, tapes), or optical media (e.g. CD-ROM, DVD), or audio materials. Additionally or alternatively, the media can contain internet addresses that provide said instructions.

The expression "reagent which allows determining the expression level of a gene" means a compound or set of compounds that allows determining the expression level of a gene both by means of the determination of the level of mRNA or by means of the determination of the level of protein. Thus, reagents of the first type include probes capable of specifically hybridizing with the mRNAs encoded by said genes. Reagents of the second type include compounds that bind specifically with the proteins encoded by the marker genes and preferably include antibodies, although they can be specific aptamers

In a particular embodiment of the kit of the invention, the reagents of the kit are nucleic acids which are capable of specifically detecting the mRNA level URI and/or the level of proteins encoded by URI. Nucleic acids capable of specifically hybridizing with URI can be one or more pairs of primer oligonucleotides for the specific amplification of fragments of the mRNAs (or of their corresponding cDNAs) of said gene.

In a preferred embodiment, the first component of the kit of the invention comprises a probe which can specifically hybridize to a URI gene mentioned above.

The term "specifically hybridizing", as used herein, refers to conditions which allow hybridizing of two polynucleotide under high stringent conditions or moderately stringent conditions.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

In the event that the expression levels of URI according to the present invention is determined by measuring the levels of the polypeptide or polypeptides encoded by said gene or genes, the kits according to the present invention comprise reagents which are capable of specifically binding to said polypeptide or polypeptides. Thus, in one embodiment, the invention relates to a kit comprising antibodies specific for the polypeptides encoded by URI gene.

The antibodies of the kit of the invention can be used according to techniques known in the art for determining the protein expression levels, such as, for example, flow cytometry, Western blot, ELISA, RIA, competitive EIA, DAS-ELISA, techniques based on the use of biochips, protein microarrays, or assays of colloidal precipitation in reactive strips.

The antibodies can be fixed to a solid support such as a membrane, a plastic or a glass, optionally treated to facilitate the fixation of said antibodies to the support. Said solid support comprises, at least, a set of antibodies which specifically recognize the marker, and which can be used for detecting the levels of expression of said marker.

Additionally, the kit of the invention may comprise reagents for detecting a protein encoded by a constitutive gene. The availability of said additional reagents allows normalizing the measurements performed in different samples (for example, the sample to be analyzed and the control sample) to rule out that the differences in the expression of the biomarkers are due to a different quantity of total protein amount in the sample more than the real differences in the relative levels of expression. The constitutive genes in the present invention are genes that are always active or being transcribed constantly and which encode for proteins that are expressed constitutively and carry out essential cellular functions. Proteins that are expressed constitutively and can be used in the present invention include, without limitation, β-2-microglobulin (B2M), ubiquitin , 18-S ribosomal protein, cyclophilin, GAPDH, PSMB4, tubulin and actin.

In a preferred embodiment, the reagents for assaying the levels of URI comprise at least 10%, at least 20%, at least 30%, at least 40%, at least 50%, at least 60%, at least 70%, at least 80%, at least 90% or at least 100% of the total amount of reagents for assaying biomarkers forming the kit. Thus, in the particular case of kits comprising reagents for assaying the levels of URI, the reagents specific for said biomarkers (i.e. antibodies which bind specifically to URI) comprise at least 10%, at least 20%, at least 30%, at least 40%, at least 50%, at least 60%, at least 70%, at least 80%, at least 90% or at least 100% of the antibodies present in the kit.

In a preferred embodiment, the kit of the invention further comprises a reagent which allows determining the NAD+ levels.

The expression "reagent which allows determining the NAD+ levels" means a compound or set of compounds that allows determining the NAD+ levels by for example, detecting the formation of the formed NADH through the reduction of a reagent.

All the terms and embodiments previously described are equally applicable to this aspect of the invention.

### 6- Use of the kit of the invention

In another aspect, the invention relates to the use of a kit of the invention for designing a customized therapy for a subject diagnosed with a liver cancer or suffering early signs of chronic liver damage, for diagnosing liver cancer or for predicting the risk of a patient suffering hepatitis to develop liver cancer according to the methods of the invention.

All the terms and embodiments previously described are equally applicable to this aspect of the invention.

Additional aspects of the invention include:
[1]-Nicotinamide riboside or a derivative thereof for use in the treatment and/or prevention of liver cancer or pancreatic cancer in a subject.
[2]- Nicotinamide riboside or a derivative thereof for use according to aspect 1, wherein the subject shows high level of expression of a gene inducing DNA damage.
[3]- Nicotinamide riboside or a derivative thereof for use according to aspect 2, wherein a gene inducing DNA damage is selected from the group consisting of URI gene and c-Myc gene.
[4]- Nicotinamide riboside or a derivative thereof for use according to any of aspects 1-3, wherein the liver cancer is hepatocellular carcinoma.
[5]- Nicotinamide riboside or a derivative thereof for use according to any of aspects 1-3 wherein the pancreatic cancer is pancreatic ductal adenocarcinoma.
[6]- Nicotinamide riboside or a derivative thereof for use according to any of aspects 1 to 5, wherein the nicotinamide riboside or a derivative thereof is administered orally.
[7]- Nicotinamide riboside or a derivative thereof for use according to aspect 6, wherein the nicotinamide riboside or a derivative thereof is administered as a nutritional supplementation.
[8]- Nicotinamide riboside or a derivative thereof for use according to any of aspects 1 to 7, wherein the subject shows early signs of chronic liver damage or pancreatic intraepithelial lesions.
[9]- Nicotinamide riboside or a derivative thereof for use according to aspect 8, wherein the early signs of chronic liver damage or pancreatic intraepithelial lesions are early signs of precancerous lesions.
[10]- Nicotinamide riboside or a derivative thereof for use according to any of aspects 1 to 9, wherein the nicotinamide riboside or a derivative thereof is administered in combination with a chemotherapeutic agent.
[11]- Nicotinamide riboside or a derivative thereof for use according to aspect 10, wherein the chemotherapeutic agent is selected from Table 1.
[12]- Nicotinamide riboside or a derivative thereof for use according to aspect 11, wherein the chemotherapeutic agent is selected from the group consisting of sorafenib, olaparib, hydroxyurea, EX-572 and combinations thereof.
[13]- An *in vitro* method for designing a customized therapy for a subject diagnosed with a liver cancer or pancreatic cancer, suffering early signs of chronic liver damage or suffering pancreatic intraepithelial lesions which comprises
   a) determining the level of DNA damage in a sample from said subject, and
   b) comparing said levels with a reference value,
   wherein an increased level of DNA damage in said sample with respect to the reference value is indicative that the subject is to be treated with nicotinamide riboside or a derivative thereof.
[14]- An in vitro method according to aspect 13 wherein the gene inducing DNA damage is selected from URI and c-Myc.
[15]- An *in vitro* method according to any of aspects 13 or 14, wherein the liver cancer is hepatocellular carcinoma.
[16]- An *in vitro* method according to any of aspects 13 or 14, wherein the chronic liver damage is caused by hepatitis.
[17]- An *in vitro* method according to any of aspects 13 or 14, wherein the pancreatic cancer is pancreatic ductal adenocarcinoma
[18]- An *in vitro* method according to any of aspects 13 to 17, wherein the nicotinamide riboside or a derivative thereof is administered orally.
[19]- An *in vitro* method for diagnosing liver cancer in a subject which comprises:
   a) determining the level of expression of URI gene in a sample from said subject, and
   b) comparing said level with a reference value,
   wherein an increased level of expression of URI gene in said sample with respect to the reference value is indicative that the subject suffers liver cancer.
[20]- An *in vitro* method for predicting the risk of developing liver cancer in a subject suffering hepatitis which comprises:
   a) determining the level of expression of URI gene in a sample from said subject, and
   b) comparing said levels with a reference value,
   wherein an increased level of expression of URI gene in said sample with respect to the reference value is indicative that the subject shows high risk of developing liver cancer.
[21]- Kit comprising a reagent which allows determining the expression level of URI gene.
[22]- Kit according to aspect 21, further comprising a reagent which allows determining the NAD+ levels.
[23]- Use of a kit according to any of aspects 21 or 22 for designing a customized therapy for a subject diagnosed with a liver cancer or suffering early signs of chronic liver damage, for diagnosing liver cancer or for predicting the risk of a patient suffering hepatitis to develop liver cancer.

The invention will be described by way of the following examples which are to be considered as merely illustrative and not limitative of the scope of the invention.

### MATERIAL AND METHODS

### Generation and Handling of Mice

hURI knock-in mouse model (ColhURI) has been generated by flippase-mediated targeting and recombination of inducible human *URI* (hURI) cDNA tagged with the FLAG peptide in the 3' untranslated region of the homing locus of the collagen type I, alpha 1 locus (*Colla1*) *Cola1* gene in KH2 embryonic stem cells (ESCs), having a tetracycline operator (Tet-op). In order to express hURI specifically in hepatocytes, ColhURI mouse has been crossed with a line containing the tetracycline-dependent transactivator (tTA) under the control of the liver activated protein (LAP) promoter to generate LAP-tTA/hURI*tetOFF* mouse, named hURI-tetOFFhep mouse. Hepatocyte-specific ectopic hURI expression can be switched off by administration of doxycycline. hURI is expressed since conception and mice were off doxycycline, unless otherwise stated (Table 2). The URI conditional knockout mouse with *uri* floxed allele was generated by homologous recombination in embryonic stem (ES) cells (URI lox). In this allele, the exon 4 of *uri* was flanked by two LoxP sites. In addition, a neomycin resistance gene (Neo) flanked by two FRT sites was inserted before the second LoxP site for drug selection and was removed by expressing Flp recombinase. Expression of Cre recombinase deletes the targeted exon 4 and generates a delta allele (URIΔ/Δ), leading of inactivation of the URI gene. The germ line transmission of the founder mouse generated through an intercross between the chimeras and the Flp-deleter strain in order to remove the neomycin cassette was checked by Southern blot (5' and 3' arms were checked) and PCR analysis. Deletion of one allele by Cre recombinase in heterozygous ES cells was verified by reduced URI expression via WB analysis (Table 2).

**Table 2. Promoters used to generate hepatocyte specific gemms**

| ***N*** | ***Promoter*** | ***Cell spec*.** | ***Onset*** | ***Genotype*** | ***Gain of funtion (relative expression level)*** | ***Notes*** |
|---|---|---|---|---|---|---|
| **1** | LAP | Hepatocytes | | LaptTA (+/T) | NA | |
| | | | Embyonic (E10.5) | (ColhURI(+/Ki);LaptTA(+/T) | 2-3 fold the endogenous (WB) | Mutant |
| | | | | (ColhURI(Ki/Ki);LaptTA(+/T) | 12-18 fold the endogenous (WB) | Homozygous Mutant |
| | | | 8 weeks | (ColhURI(+/Ki);LaptTA(+/T) | 2-3 fold the endogenous (WB) | Mutant (Adult stage) |
| **2** | SA-Cre-ERT2 | Hepatocytes | | SAcreERT2 (+/Ki) | Loss of funtion (% of deletion) | |
| | | | 3 weeks | (URI(f/f);SAcreERT2 (+/Ki) | 80-90 % (IHC and WB) | URI(D/D)*hep* |
| | | | | (URI(f/+);SAcreERT2 (+/Ki) | | URI(+/D)*hep* |

Homozygous (lox/lox) and heterozygous (+/lox) mice carrying the URI lox allele were viable, fertile, and exhibited no overt abnormalities. Crossing URI (+/lox) heterozygous mice with a Mox-Cre line, to deplete URI ubiquitously, generated viable URI (+/Δ) mice (Δ/Δ homozygotes obtained from intercrosses were non-viable). p53 inactivation in the hURI-tetOFFhep mouse was performed by crossing hURI-tetOFFhep mice with p53ERTAM mice, in which p53 activation requires ectopic 4-hydroxytamoxifen provision. All mice used in this study have been backcrossed to C57BL/6 mice for at least 7 generations. All mice were housed in specific pathogen-free (SPF) animal facility Unit of CNIO. All experiments were approved by the CNIO-ISCIII Ethics Committee for Research and Animal Welfare (CEIyBA) and performed in accordance with the guidelines for ethical conduct in the care and use of animals as stated in the international guiding principles for biomedical research involving animals, developed by the Council for International Organizations of Medical Sciences (CIOMS). Mice were kept in close observation and sacrificed when they displayed signs of sickness, in accordance to the Guidelines for humane endpoints for animals used in biomedical research. Littermates were always used as controls. Food (Harlan Laboratories) and water were provided *ad libitum.* Same experiments have been performed by sacrifying mice at the same time and same lobes were used for the same experiment.

### Human Samples

Human samples were obtained from the histopathology files of University College Hospital, University College London (UCL), from patients after approval by the Institutional Research Ethics Committee (Central London REC 3, Reference 06/Q0512/106). The construction and analysis of a tissue microarray of HCC was approved by the appropriate ethical committee.

### Antibodies

Antibodies recognizing hURI and mURI have been described previously (Djouder et al., 2007 cited at supra). Ki-67 (1:1) and Cyclin D1 (1:1000) were purchased from Dako. MAD2 (1:250), PCNA (1:1000), α-SMA (1:1000) and p65 (1:1000) were purchased from Santa Cruz. phospho-p44/42 ERK (Thr202/Tyr204) (1:1000), ERK (1:1000), S6K1 (1:1000), phospho- S6 (Ser240/244) (1:1000), S6 (1:1000), phospho-p38 (Thr180/Tyr182) (1:1000), p38 (1:1000), phospho-STAT3 (Tyr705) (1:1000), STAT3 (1:1000), phospho-AKT (Ser473) (1:1000), AKT (1:1000), BAX (1:1000), phospho-p53 (Ser18) (1:500), p53 (1:500), acetylp53 (Lys379) (1:500), cleaved caspase3 (1:500), phospho-Chk1 (Ser345) (1:500) and HSP90 (1:1000) were from Cell Signaling Technology. Phospho-H2AX (Ser139) (1:1000) was purchased from Merck Millipore. Phospho-Chk2 (Thr68) (1:500), Chk1 (1:500), Chk2 (1:500) were purchased from Upstate (Millipore). Phospho-S6K1 (Thr389) (1:1000), and GAPDH (1:2000) were from Abcam. Flag antibody (1:1000) was from Agilent. JNK1 (1:1000), p21 (1:1000) and PARP (1:500) was from BD-Pharmingen. Vinculin (1:1000) was purchased from Sigma. TDO2 (1:1000), AFMID (1:500), GCDH (1:500), CPS1 (1:1000), GNMT1 (1:1000) and NAMPT (1:1000) were purchased from Proteintech. AhR (1:1000) was from Enzo life sciences and ER (1:1000) was from Bethyl laboratories. AFP (1:500) was from R&D systems.

### Cell Culture and siRNA

Knockdown experiments were performed using ON-TARGET plus SMART pool siRNA targeting human AhR, ER, TDO2, AFMID and URI as well as control siRNA, and were purchased from Dharmacon. HepG2, Huh-7, SNU398, SNU449 and HEK-293T Cells were grown in complete DMEM medium supplemented with 10% fetal calf serum and 100 units/ml penicillin and 0.1 mg/ml streptomycin purchased from Gibco. siRNA were transfected using Lipofectamine-RNAiMAX according to the manufacturer's instructions.

### Immunoblotting and Immunoprecipitation

For immunoblotting, liquid N2 snap frozen liver tissues were used to prepare lysates. 50 to 100 mg of tissues were lysed using RIPA lysis buffer containing 10 mM Tris pH 7.5, 100 mM NaCl, 1 mM EDTA, 1 mM EGTA, 1 mM NaF, 20 mM Na4P2O7, 2 mM Na3VO4, 0.1% SDS, 0.5% sodium deoxycholate, 1% Triton-X 100, 10% glycerol and supplemented with 10 mg/ml proteases inhibitor aprotinin and 1mM PMSF followed by homogenization using Precellys 24 Bead Mill homogenizer (Bertin Technologies) (15 x 2 s, power set to 5500 w) and then clarified by centrifugation at 4°C and 10.000 g for 10 min. Protein concentration was measured by using Bio-Rad Bradford reagent (Bio-Rad) and bovin serum albumin (BSA) as standard protein. 1mg/ml concentrated lysates were made by boiling the appropriate amount of protein lysates with 2X laemmli buffer (4% SDS, 20% glycerol, 10% 2-mercaptoethanol, 0.004% bromophenol blue in 0.2 M Tris-HCL of pH 7) at 70°C, for 10 min. 10-30 µg of protein lysates were subjected into SDS-PAGE gels, and transferred to nitrocellulose membranes. The membranes were blocked with 5% nonfat milk in Tris-buffered saline containing 1% Tween 20 for Ih at room temperature. Blots were immune-stained with indicated antibodies. Immunoblots were processed by ECL (Amersham) according to the manufacturer's instructions. For immunoprecipitation, samples and assays were performed as described previously (Djouder et al., 2007 cited at supra).

### Chromatin Immunoprecipitation (ChIP)

For Chomatin Immuno-precipitation, 6.107 SNU449 cells were cross-linked for 10 min at RT in 1% formaldehyde. Cross-linking reactions were stopped by adding 1.25 M glycine to a final concentration of 125 mM. Cells were centrifuged for 10 min at 4°C and then washed in cold PBS. Cells were lysed with 1 mL of lysis buffer-1 (50 mM HEPES-KOH, pH 7.5, 140 mM NaCl, 1 mM EDTA, 10 % glycerol, 0.5 % NP-40, 0.25 % Triton X-100, 1 X protease inhibitor) followed by centrifugation at 4°C and resuspension of the pellet in lysis buffer-2 (10 mM Tris-HCl, pH8.0, 200 mM NaCl, 1 mM EDTA, 0.5 mM EGTA, 1 X protease inhibitors. Finally the pellets were resuspended in 1 mL of lysis buffer-3 (10 mM Tris-HCl, pH8.0, 100 mM NaCl, 1 mM EDTA, 0.5 mM N-lauroylsarcosine, 1 X protease inhibitors), and 100 µl of 10 % Triton X-100 were added and sonicated for 20 min in a Covaris sonicator.

The soluble fraction was quantified with Bradford, and 400 µg was used to immunoprecipitate the transcription factors and IgG's used as a control. Chromatin and
antibody mixtures were incubated overnight at 4°C in total volume of 500 µl.

Immunoprecipitated mixture was washed with a low salt buffer (20 mM Tris-HCl, pH 8.0, 150 mM Nacl, 2 mM EDTA, 0.1 % SDS and 1 % Triton X-100), followed by high salt buffer (20 mM Tris-HCl, pH 8.0, 500 mM NaCl, 2 mM EDTA, 0.1% SDS and 1 % Triton X-100) and finally with LiCl wash buffer (10 mM Tris-HCl, pH 8.0, 250 mM LiCl, 1 mM EDTA, 1 % Na-Deoxycholate and 1 % NP-40). Samples were decross-linked by resuspending the beads in 210 µl of elution buffer (50 mM Tris-HCl, pH 8.0, 10 mM EDTA and 1 % SDS), incubating at 65°C for 15 min and treating with 2.5 µl (from 32 mg/mL) of RNAse A for 2 hours. Next, 4 µl of (20 mg/ml of stock) of Proteinase K was added and incubated at 55°C for 2 hrs. DNA was extracted by phenol/chloroform/isoamylalcohol mixtures, washed with 80% EtOH and resuspended in 200 µl of TE buffer. This was followed by quantitative real-time PCR. The specific primers used are listed below. P1-TDO2-F (AGGAGACTACACTACTGGGAGAAG) (SEQ ID NO:1); P1-TDO2-R-(CTTGTTTGCTAGCAGGGCGT) (SEQ ID N0: 2); P4-TDO2-F (TTGTCTATGGGCAGGGTGAT) (SEQ ID NO:3) ; P4- TDO2-R (GCCTGGTGCGAAAAACGAG) (SEQ ID NO:4); P5-TDO2-F CTCCTGTAAGGACCTACCTAGC) (SEQ ID NO:5); P5-TDO2-R (ACCAAACTCCTCTGGCGTATC) (SEQ ID NO:6); P6-AFMID-F (CTAGAGGCTAGCAGCAGTGTG) (SEQ ID NO:7); P6-AFMIDR (CTTCCAGATCGCCCAGTACA) (SEQ ID NO:8); P7-AFMID-F (CAGGCACAGTTGGATCTCT) (SEQ ID NO:9); P7- AFMID-R (AGAGGACAGCCTCGGGTTAT) (SEQ ID NO:10); P8-AFMID-F (GCGGGTGTATGGTTACCTGT) (SEQ ID NO:11); P8-AFMID-R (ATGCCCGAGTGTTGTGTTGT) (SEQ ID NO:12); P9- AFMID-F (GCCTCAGTAAATGGGTTGAGAGA) (SEQ ID NO:13); P9-AFMID-R (CTGCACGCTACTTGCTTGTC) (SEQ ID NO14); P10-AFMID-F (CACTGAGGCCAAGGCTAGAG) (SEQ ID NO:15); P10-AFMID-R (AGGAACACCATGGACGCATT) (SEQ ID NO:16); P11-AFMID-F (TAAAAGCAGCCCAATGCGGG) (SEQ ID NO:17); P11-AFMID-R (GTCGACCTCTGACCTCCACAT) (SEQ ID NO:18), Chr3-intron-F (CTTGGCCCTTCCTCTCCTAA) (SEQ ID NO:19); Chr3-intron-R (TGCAGTGGTCAGAAGATGTGT) (SEQ ID NO:20).

### Immunohistochemistry and Immunofluorescence

Freshly harvested murine livers were fixed immediately in 10% buffered formalin solution overnight and embedded in paraffin. Sections of 3 µm were deparafinized, rehydrated and antigen retrieved by using 1M sodium citrate buffer (pH 6.5). After blocking endogenous peroxidase using 3% H2O2 (in methanol) for 5 min, sections were then blocked with 1:200 goat serum 5%BSA/PBST for Ih at RT. Furthermore, sections were incubated with primary antibodies overnight at 4°C, after which Vectastain ABC kit (Vector Laboratories, Inc) was used, following manufacturer's instructions. Sections were then incubated with 33-diaminobenzidinetetracloride (DBA) and counterstained with hematoxylin. In the case of immunofluorescence, sections were incubated with PE-conjugated secondary antibodies (1:500) for Ih at RT, followed by DAPI staining. Quantification was performed either by counting the number of positive cells in a minimum of six 10X or 20X microscopic fields, or the percentage of positive area using Color Deconvolution plug-in in Image J.

### Cytoplasmic Fractionation

For cytoplasmic extracts, livers were homogenized with the cytoplasmic buffer (10 mM Hepes pH 7.4, 3 mM MgCl2, 40 mM KCl, 5 % Glycerol, 0.5% NP-40, 2 mM DTT) supplemented with protease inhibitors. Pellets were incubated on ice 5 min and centrifuged for 5 min at 1500 rpm at 4°C. The supernatant fractions correspond to the cytoplasmic fractions. Protein concentrations of the fractions were determined using Bradford assay (BioRad) and equal amounts of each fraction were subjected for SDS-PAGE and blotted to nitrocellulose membranes for Western blotting with the indicated proteins.

### Genotyping and qRT-PCR

For genotyping tail DNA was extracted by overnight incubation of tails with the following buffer (1% SDS, 0.1M NaCl, 0.1M EDTA and 0.05M Tris (pH8)). Extracted DNA was precipitated using ice-cold isopropanol which was further washed with 70% ethanol. The DNA pellet was further dried and resuspended in 400 µl of water. 1 µl of DNA was used for genotyping. For the *ColhURI* locus the following primers were used: P1-F: GCACAGCATTGCGGACATGC (SEQ ID NO:21); P2-R: CCCTCCATGTGTGACCAAGG (SEQ ID NO:22); P3-R: GCAGAAGCGCGGCCGTCTGG (SEQ ID NO:23). For the *LaptTA* transactivator locus the following primers were used: P1-F: TCTGAGCATGGCCTCTAA (SEQ ID NO:24); P2-R: GCTGGAGTAAATTTCACAGTG (SEQ ID NO:25); P3-R: TCTCACTCGGAAGGACAT (SEQ ID NO:26). For qRT-PCR, total RNA was extracted from 20-50 mg snap frozen liver tissue, using Trizol (Sigma). First strand cDNA synthesis was performed by using Ready-to-go first strand beads (GE Healthcare). qRT-PCR was performed using an ABI PRISM 7700 (Life Technologies), and GoTaq Real-Time qPCR mix (Promega). Fold changes have been determined by using 2- ΔΔCT (Livak and Schmittgen, 2001, Method. Methods 25, 402-408) and normalized to β-actin. Finally, the fold changes were obtained by converting the logarithmic scale to an exponential scale (2^ΔΔCT). The following primers were used β-Actin-F: CACAGCTGAGAGGGAAATCG (SEQ ID NO:27). β-Actin-R: AGTTTCATGGATGCCACAGG (SEQ ID NO:28). Timp1-F: AGGTGGTCTCGTTGATTCGT (SEQ ID NO:29). Timp1-R GTAAGGCCTGTAGCTGTGCC (SEQ ID NO:30). α-SMA-F: CAATGGCTCTGGGCTCTGTA (SEQ ID NO:31). α-SMA-R: TCATCCCCCACATAGCTGTC (SEQ ID NO:32). Colla-F: AGGGCCAGGGGGTCCAGCATTTC (SEQ ID NO:33). Colla1- R: GGTGCCCCCGGTCTTCAG (SEQ ID NO:34). Bax-F: GTGAGCGGCTGCTTGTCT (SEQ ID NO:35). Bax-R: GGTCCCGAAGTAGGAGAGGA (SEQ ID NO:36). XIAP-F: GAGGCAGGAAGCTAACGTTTT (SEQ ID NO:37). XIAP-R: GCAGCTCGTCTGAGATCAATG (SEQ ID NO:38). PUMA-F: CTGTATCCTGCAGCCTTTGC(SEQ ID NO:39), PUMA-R: ACGGGCGACTCTAAGTGCT (SEQ ID NO:40).

### Proteomic Analysis

Liver tissues were extracted and proteins were digested using a modified FASP protocol. Peptides were labeled with iTRAQ reagents and samples were pooled. Then, the complex mixture was subjected to IEF fractionation. The resulting fractions were separated by on-line nano-LC and analyzed by electrospray MS/MS using a LTQ Orbitrap Velos mass spectrometer (Thermo Scientific). Raw files were searched against UniProtKB/Swiss-Prot mouse database (release date: October 19, 2011; 16407 entries) using MASCOT as a search engine through the Proteome Discoverer (Thermo Scientific) software. Peptides were filtered at 1% FDR using a concatenated database.

### iTRAQ

For protein extraction, 20 mouse livers (5 per group) were individually processed. Lysis solution (4% SDS, 10 mM DTT, 100 mM HEPES, pH=7.6) including 0.1% Benzonase Nuclease (Novagen) plus a cocktail of protease inhibitors was added to small pieces of tissue (1 mg tissue: 20 µL buffer). Samples were homogenized in a Precellys 24 Bead Mill Homogenizer (Bertin Technologies) (15 x 2 s, power set to 5500 w) and then clarified by centrifugation at 4°C and 16000 rpm for 15 min. Recovered supernatants were cleaned-up by methanol-chloroform extraction (Kline et al., 2008 J Proteome Res 7, 5055-5061) and pellets were dissolved in 7M urea 2M tiourea 0.1M TEAB by sonication and vortexing. The protein concentration of the samples was determined according to the Bradford assay using BSA as standard (Protein Assay Kit, Bio-Rad). Samples for each group were prepared by pooling an equal amount of protein from each individual

### Protein Digestion and Labelling with iTRAQ Reagents

Samples were digested using the filter aided sample preparation (FASP) method (Wisniewski et al., 2009, Nat Methods 6, 359-362) with some modifications. Briefly, 100 ug of each sample dissolved in 7M urea 2M tiourea was loaded on the filter, reduced with 10 mM DTT for 1 h at 37°C and alkylated using 50 mM iodoacetamide for 20 min in the dark. The excess of reduction and alkylation reagents was washed. The proteins were digested overnight at RT using endoproteinase Lys- C from *Acromobacter lyticus* M497-1 (Wako Pure Chemical Industries) with 1:50 enzyme to protein ratio. Finally, trypsin (Promega) was added and samples were subjected to a second digestion for 6 h. Each tryptic digest was labeled according to the manufacturer's instructions (ABSciex) with one isobaric amine-reactive tag as follows: liver peptides from 1 week control mice were tagged with Tag114. Tag115 was used for 8 week control mice. Tag116 was used for 8 week mutant mice and Tag117 for 1 week mutant mice. After 1 h incubation, labeled samples were pooled, and evaporated to dryness in a vacuum centrifuge. The iTRAQ sample was cleaned up using a Sep-Pak C18 cartridge for SPE (Waters Corp) (Ernoult et al., 2008 Proteome Res 6, 821-827). Eluted peptides were vacuum-dried and reconstituted in OFFGEL solution (5% glycerol, 1% ampholytes pH 3-10) prior to electrofocusing.

### iTRAQ Data Analysis

The raw files were processed using the Proteome Discoverer 1.3.0.339 software suite (Thermo Scientific). The fragmentation spectra were searched against the UniProtKB/Swiss- Prot mouse database (released date: October 19, 2011; 16407 entries) using MASCOT (Perkins et al., 1999, Electrophoresis 20, 3551-3567) as the search engine (v 2.2) with the precursor and fragment mass tolerances set to 10 ppm and 0.075 Da, respectively, and with up to two missed cleavages.Lysine and peptide N-termini labeling with iTRAQ-4plex reagent as well as carbamidomethylation of cysteine were considered as fixed modifications, while oxidation of methionine was chosen as variable modification for database searching. Peptides identification was filtered at 1% false discovery rate (FDR) and thus not dependent on the peptide score. Only peptides with high confidence were considered. The results were then exported into Excel for manual data interpretation. Although relative quantification and some statistical data were provided by the Proteome Discoverer software, an additional 1.3-fold change cut-off for all iTRAQ ratios (ratio <0.77 or >1.3) was selected to classify proteins as up- or down-regulated (Chen et al., 2007, Mol Cell Proteomics 6, 2072-2087; Gan et al., 2007, J Proteome Res 6, 821-827; Ho et al., 2009, J Proteome Res 8, 583-594). Proteins with iTRAQ ratios below the low range (0.77) were considered to be under-expressed, while those above the high range (1.3) were considered overexpressed. Only proteins identified with 2 or more unique peptides and at least 3 iTRAQ peptide counts were considered. Gene Ontology analyses of the identified proteins were performed.

### OFFGEL Fractionation

For pI-based peptide separation, we used the 3100 OFFGEL Fractionator system (Agilent Technologies) with a 24-well set-up. The IPG gel strips of 24 cm-long (GE Healthcare) with a 3-10 linear pH range were rehydrated for 15 min with the Peptide IPG Strip Rehydratation Solution according to the protocol of the manufacturer. Subsequently, 150 µL of sample was loaded in each well. Electrofocusing of the peptides was performed at 20°C and 50 µA until the 50 kVh level was reached. After focusing, the 24 peptide fractions were withdrawn and the wells rinsed with 100 µL of a solution of 0.1%TFA. Rinsing solutions were pooled with their corresponding peptide fraction. All fractions were evaporated by centrifugation under vacuum. Solid phase extraction and salt removal was performed with home-made columns based on Stage Tips with C8 Empore Disks (3M) (Rappsilber et al., 2003, Anal Chem 75, 663-670.) filled with Poros Oligo R3 resin (Life Technologies). Eluates were evaporated to dryness and maintained at 4°C. Just prior nano-LC, the fractions were resuspended in 0.1% formic acid (FA).

### Peptide Analysis by NanoLC-MS/MS

Digested samples were separated by on-line reversed-phase nanoscale capillary LC and analyzed by electrospray MS/MS. The experiments were performed on an Eksigent nano LC system (Eksigent technologies) coupled to an LTQ Orbitrap Velos mass spectrometer (Thermo Scientific) equipped with a nanoelectrospray ion source (Proxeon Biosystems). Peptides were loaded from a cooled nanoLC AS-2 autosampler (Eksigent). In order to pre concentrate and desalt the samples before switching the pre-column in line with the separation column, 5 µL from each sample was loaded onto a reversed-phase ReproSil Pur C18-Aq 5 µm 0.3 x 10 mm trapping cartridge (SGE Analytical, Victoria, Australia), and washed for 10 min at 2.5 µL/min with loading buffer (0.1% FA). The peptides were eluted from a RP ReproSil Pur C18-AQ 3 µm 200 x 0.075 mm (Dr. Maisch GmbH) by application of a binary gradient consisting of 2% ACN in 0.1% FA (buffer A) and 100% ACN in 0.1%FA (buffer B), with a flow rate of 300 nL/min. Peptides were separated using the following gradient: 0-5 min 2% B, 5-150 min 60% B and 150-165 min 98% B. The column was operated at a constant temperature of 30°C. The LTQ Orbitrap Velos was operated in positive ionization mode. The MS survey scan was performed in the FT analyzer scanning a window between 250 and 1750 m/z. The resolution was set to 60 000 FWHM at *m*/*z* 400. The *m*/*z* values triggering MS/MS with a repeat count of 1 were put on an exclusion list for 60 s.

The minimum MS signal for triggering MS/MS was set to 1000 counts. In all cases, one microscan was recorded. The lock mass option was enabled for both MS and MS/MS mode and the polydimethylcyclosiloxane ions (PDMS, protonated (Si(CH3)2O))6; m/z 445.120025) were used for internal recalibration of the mass spectra (Olsen et al., 2005, Mol Cell Proteomics 4, 2010-2021). For the HCD, up to the 15 most abundant isotope patterns with charge ≥2 from the survey scan were selectedwith an isolation window of 2 m/z fragmented in the C-trap collision cell. Normalized collision energy was set to 42%, the Q value to 0.25 and an activation time to 0.10 ms.Waveform filter was activated. The resulting fragments were detected in the Orbitrap system with a resolution of 7500 FWHM at m/z 400. The maximum ion injection times for the survey scan and the MS/MS scans were 500 ms and 250 ms respectively and the ion target values were set to 1E6 and 5E4, respectively for each scan mode. The 24-fractions were run in duplicates.

### RNA Extraction and Sequencing

For RNA extraction, 20 livers (5 per group) were individually extracted. RNA Integrity Numbers were 8.3 on average (range 7.5 to 9.2) when assayed by Lab-chip technology on a 2100 Bioanalyzer (Agilent). Sequencing libraries were prepared as in "TruSeq RNA Sample Preparation Guide" (Part # 15008136 Rev. A) with final PCR amplification limited to 10 cycles. The resulting purified cDNA libraries were sequenced on a Genome Analyzer IIx, following manufacturer's protocols. Samples were analyzed in different paired end sequencing runs. In order to balance read length and adjust base calling quality within conditions, reads from 8-week samples were trimmed to 50 bases. Likewise, reads from 1-week samples were adjusted to 78 bases. Reads were aligned to the mouse genome (NCBI37/mm9) with Tophat (Trapnell et al., 2012 Nat Protoc 7, 562-578) version 1.3.1 (including Bowtie 0.12.7 (Langmead et al., 2009, Genome Biol 10, R25) and Samtools version 0.1.16 (Li et al., 2009, Bioinformatics 25, 2078-2079) allowing a maximum of 2 mismatches and 5 multihits for each read. The RNA fragment length was 230bp on average. Transcripts were assembled and their abundances estimated with Cufflinks version 1.0.3 (Trapnell et al. cited at supra). After that, we performed a differential expression analysis with Cuffdiff 1.0.3.

### Rapamycin Treatment

Rapamycin (Sirolimus) was purchased from TOKU-E biosciences (R001) and stored at 4°C. Rapamycin diet was prepared by Harlan laboratories to a final concentration of 14 mg/kg of food. Mice were feed 2.24 mg/kg of body weight per day as previously described (Menon et al., 2012, Sci Signal 5, ra24.).

### 3,5-diethoxycarbonyl-1,4-dihydrocollidine (DDC) Treatment

DDC was mixed with chow diet to a final concentration of (0.5% w/w) by Harlan laboratories. Mice were fed ad libitum of DDC diet, according to the protocol.

### Diethylnitrosamine (DEN) Treatment

14 days old mice were injected intraperitoneally with 25mg/kg of diethylnitrosamine (DEN) (Sigma) according to the previously described protocol (Vesselinovitch and Mihailovich, 1983, Cancer Res 43, 4253-4259). In experiments using genetic ablation of URI, consecutive to DEN injections, mice were fed with tamoxifen diet since weaning for the next 2 weeks, then changed to chow diet. Mice were sacrificed after 24 weeks of age and tumorigenesis was assessed macroscopically and quantified.

### NAD+ Determination

NAD+ levels were determined using a commercial kit (Enzychrom, BioAssays Systems, CA), and as previously described (Canto et al., 2012, Cell Metab *15*, 838-847). NAD+/NADH assay kit is based on a lactate dehydrogenase cycling reaction, in which the formed NADH reduces a formazan (MTT) reagent.

### PARP Activity

PARP activity was measured from the liver lysates using TRIVIGEN colorimetric assay (4677-096-K). In brief, fresh livers were lysed in PARP buffer, and 10 µg of the protein from the supernatant was used. 96 well plate coated with histones were rehydrated and the sample mixtures were prepared using 10 µl of protein lysate, 15 µl of water and 25 µl of PARP cocktail (biotinylated NAD+). Additionally, protein standards were also added to the wells by diluting 25 µl standards and 25 µl PARP cocktail. After washing with PBS and 0.1 % Triton X-100 followed by 2 washes of PBS, diluted strep-HRP was added and incubated for 60 min.

Finally, color was developed by adding TACS-Sapphire colorimetric substrate and reading the absorbance at 450 nm.

### Metabolic Labeling

Huh7, HepG2, SNU398 and SNU449 cells were transfected with siCtr or siAFMID, grown in 12 well plate until they reached 60% confluence. Cells were starved for 5 hours for tryptophan in tryptophan free media. 2.5 mM of [*benzene-ring-U-*14C]-tryptophan was provided to the cells and incubated at 37°C for 5 hours. Media was removed and the cells were washed 3 times with cold PBS. Metabolites were extracted in methanol and water (80:20) mixture, incubated for 10 mins at 4°C. Metabolic lysates were centrifuged at maximum speed for 20 mins, at 4°C. Radiolabelled samples were separated by thin layer chromatography (TLC) using ammonium acetate (1M, pH5) and ethanol (30:70). Cellulose F plates were used to separate the metabolites. Labelled NAD+-[carbonyl-14C] was used as positive control to calibrate the relative migration of labelled metabolites. After the migration was finished, TLC plates were dried and exposed to a radiosensitive PhosphorImager screen.

### Ro-61-8048 Treatment

The compound was purchased from Sigma-Aldrich (SML0233), and dissolved in pure DMSO for a final concentration of 593 mM. 9 week old in house C57BL/6 mice were given DDC diet for 4 days and then shifted to chow diet. Mice were then injected with either RO-61-8048/Sunflower Seed Oil (25mg/Kg) or DMSO/Sunflower Seed Oil (1:100) intraperitoneally for 3 consecutive days.

### Nicotinamide Riboside Treatment

Nicotinamide riboside (97% purity) was purchased from Waterstonetech Pharma (Indianapolis, USA) and stored at -80°C. 500 mg/kg/day of NR was dissolved in ice cold water and immediately mixed thoroughly with cold amorphous chow diet purchased from Harlan Laboratories. Pelleted NR food was stored at -20°C. Mice were fed with NR diet ad-libitum as indicated in the experiment. Fresh food was prepared every week.

### Statistical Analysis

Statistical analyses were performed using GraphPad Prism V5.0 software. Statistical significance (p) (p < 0.05 = *, p < 0.001= **, p<0.0001= ***) between the means of a minimum of three groups was determined using unpaired two-tailed Student's t test. Results are expressed as the mean value +/- Standard Deviations (SD). All results are representative of at least three independent experiments. Kaplan Meier curve for the survival analysis of mice was calculated using 17 control and 17 mutant mice. Statistical parameter false discovery rate (FDR) estimates the probability of a gene set with false positive finding. Normalized enrichment score (NES) allows to compare enrichment analysis results across gene sets. Cox regression and survival analysis were performed for target genes in human HCC datasets, using SPSS software (v20).

### ACCESSION NUMBERS

The proteomic data obtained in this study have been deposited to the ProteomeXchange Consortium (http://proteomecentral.proteomexchange.org) via the PRIDE partner repository with the dataset identifier PXD000296 (Vizcaino et al. Nucleic Acids Res 41, D1063-1069). RNA sequencing data obtained in this study have been deposited under GEO accession number GSE48654. Reviewers can access the data via private access link (http://www.ncbi.nlm.nih.gov/geo/query/acc.cgi?token=ubstgkoqtpmdpub&acc=GSE48654).

### EXAMPLES

### Example 1-URI Expression in Hepatocytes Induces Spontaneous Liver Tumors and Recapitulates Human HCC

To elucidate primary events in HCC development, the inventors generated a *Colla1* knock-in mouse, expressing hURI via a tetracycline-dependent transactivator, controlled by the hepatocyte-specific liver activated protein (LAP) promoter. These mice (designated hURI-tetOFFhep) and littermates lacking hURI expression are referred to hereafter as "mutants" and "controls", respectively. Without doxycycline, hURI was expressed specifically in hepatocytes from one allele from E10.5, roughly twice as strongly as mouse URI (Figure 1A), corresponding to the fold-increase of URI expression in human HCC (see below).

The inventors observed no pathological signs in 3-week-old mutants. In 8-week-old mutants they detected no tumors visually, but H&E staining revealed anisokaryotic clusters (Figure 1B) resembling low-grade dysplastic nodules observed in human hepatitis. At 12 weeks the clusters developed into high-grade dysplastic nodules (Figure 1B), reminiscent of human large liver cell dysplasia. Increasing signs of fibrosis (increases in Sirius Red staining and fibrotic markers detected by qRT-PCR) were observed in mutants' livers from 8 weeks. Between 24-54 weeks macroscopic lesions including adenoma and early HCC emerged, between 54-65 weeks low-grade and differentiated HCC were fully apparent, and between 65-75 weeks 60% of mutants developed macroscopic high-grade tumors occupying 20-50% of their livers (Figures 1B and 1C). The proliferation rate indicated that 25-50% of hepatocytes were Ki67-positive, suggesting aggressive tumors. According to World Health Organization criteria (WHO, 2008), all tumors were well/moderately differentiated: 20% glandular/acinar, indicative of telangiectatic variants, and 80% trabecular. No cholangiocarcinoma were detected (Figure 1C). Serum glucose, alanine aminotransferase and total bile acids indicated that the mutant livers were functionally damaged. Surprisingly, serum albumin levels were increased, suggesting that the liver function might not be fully compromised.

All mutants died at ∼ 85 weeks, before complete liver failure, when control mice were still apparently healthy (Figure 1D). Immunohistochemistry (IHC) and pathological analysis also revealed hURI-positive hepatocyte-like cells in 30% of mutant lungs with HCC, indicating aggressive metastases. Histopathological characterization confirmed the presence of heterogeneous tumors, of various types and grade, with collapsed reticulin fibres suggesting increases in hepatocyte death and compensatory proliferation, as indicated by Ki67 (Figure IE). Increases in alpha fetoprotein levels, a clinical marker for human HCC, varied but all tumors displayed dramatic increases in p53 phosphorylation and abundance (Figures 1E and 1F).

Fully developed HCC appeared at 30 weeks in hURI-tetOFFhep mice treated with the hepatocarcinogen diethylnitrosamine (DEN). When hURI was expressed from two alleles, increasing its expression to 6-fold compared to levels in heterozygous hURI-tetOFFhep mice, HCC were consistently detected at 10 weeks, highlighting the importance of URI dosage. Embryonic development was not involved because liver tumors (with similar incidence and features to those induced by hURI expression from E10.5) were detected in mice kept on doxycycline until 8 weeks (expressing hURI from 8 weeks) then transferred to normal chow. Thus, hURI expression in mouse hepatocytes recapitulates many clinical stages of aggressive human HCC.

### Example 2-URI is Oncogenic and Essential for Hepatocarcinogenesis

Ceasing hURI expression in 8-week-old mutants displaying dysplastic lesions for 24 weeks did not affect liver-to-body weight ratios, but reduced signs of fibrosis (Sirius Red staining and α-SMA abundance) and abolished signs of dysplastic foci and tumors (Figures 2A-2D). S6K1 activity was increased in 24-week-old mice, but remained constant when hURI expression ceased, indicating that mTOR/S6K1 activation was hURI-independent (Figure 2B). Similarly, when hURI was expressed for 24 weeks, during which macroscopic high-grade dysplastic nodules and adenomas were apparent, then switched off for 28 weeks residual anisokaryotic clusters were still detected, but no adenomas or HCCs. Thus, maintenance of preneoplastic lesions and tumor development requires continuous hURI expression.

Next, the inventors induced liver damage (which can initiate HCC) in URI loss-of-function GEMMs. To delete URI specifically in hepatocytes, they crossed URI (lox/lox) and serum albumin (SA)-CreERT2 mice, obtaining pups in expected Mendelian ratios. URI deletion in hepatocytes after tamoxifen treatment to obtain URI(+/+)*hep*, URI(+/Δ)*hep* or URI(Δ/Δ)*hep* mice was confirmed by IHC and Western blotting (WB) (Figures 2E and 2F). Because homozygous deletion in URI(Δ/Δ)*hep* mice led to organ failure and death after 10 days, URI(+/+)*hep* and heterozygous URI(+/Δ)*hep* mice, in which URI expression was approximately halved (Figures 2E and 2F), were supplied a liver damage-inducing 3,5-diethoxycarbonyl-1,4-dihydrocollidine (DDC)-supplemented diet. URI(+/Δ)*hep* mice presented significantly less liver damage and fibrosis than URI(+/+)*hep* mice, suggesting that URI reduction inhibits progression to HCC. Similarly, DEN-treatment increased URI in HCC derived from C57BL/6 mice and induced early (24 weeks) tumor development in 60% of URI(+/+)*hep* mice, but no HCC in heterozygous URI(+/Δ)*hep* mice (Figures 2G and 2H), indicating that HCC development requires URI.

### Example 3-URI-Induced DNA Damage Precedes Precancerous Lesion Formation

Phosphorylation of histone H2AX (γH2AX), a DNA damage marker, and chromosomal instability are the most convincing clinical prognostic feature of human hepatocarcinogenesis. γH2AX and p53 phosphorylation and abundance, did not differ between 1-week-old mutant and control livers. At 3 weeks, a non-pathological stage with no dysplastic lesions, both γH2AX-positive nuclei abundance and phosphorylation of the 32 kDa subunit of replication protein A at Ser-4 and Ser-8 were substantially higher in mutants, indicating replicative stress and DDR manifested by increases in p53 abundance and phosphorylation (Figures 3A-3C). Thus, hURI expression induces replication stress-dependent DDR before precancerous lesion formation, then p53-dependent apoptosis occurs in cells that unsuccessfully repair DNA (detected by cleaved caspase 3; Figure 3C). At this stage the hepatocyte proliferation rate was reduced in mutants, suggesting that DNA damage is not due to oncogene-induced high proliferation. The compensatory proliferation of neighboring cells maintaining tissue homeostasis and described to promote HCC development may not be an immediate consequence of cell death and thus, an early event driving liver tumorigenesis.

γH2AX-positive nuclei were also more abundant in older mutant livers (8- and 12-weeks-old) with obvious dysplastic lesions. Furthermore, Ser-345 phosphorylation (and hence activation) of Chk1 was enhanced in mutants, but not Thr-68 phosphorylation of Chk2, confirming the presence of SSB (Figures 3D-3F). We also detected accumulation of the Chk1 target p53 in 8-week-old mutants and several p53-stabilising changes: enhanced p53 phosphorylation at Ser-18 and acetylation at Ac-lys379 (Figure 3F), and expression of p19ARF.

Senescence-associated β-galactosidase activity, expression of several p53 target genes (*p21* and the pro-apoptotic genes Bcl-2-associated X protein, *Bax* and *p53*-upregulated modulator of apoptosis, *Puma*), and BAX protein abundance were also higher in 8- and 12-week-old mutants than in controls, while expression of X-linked inhibitor of apoptosis was decreased (Figure 3F). Accordingly, the inventors detected increases in hepatocyte death, manifested by collapsed reticulin fibres, followed by activation of compensatory proliferation mechanisms, demonstrated by increased abundance of proliferating cell nuclear antigen (PCNA), cyclin D1 and Ki67-positive nuclei, and abundance of mitotic arrest deficient 2 (MAD2), a CIN marker and downstream effector of cyclin D1 (Figure 3F), suggesting that hURI expression impaired genome integrity. Finally, inactivation of p53 in hURI-tetOFFhep mice significantly reduced survival and accelerated liver tumorigenesis (Figure 3G). 80% of the mice displayed aggressive HCC (Figure 3H), but deletion of *p19ARF* did not modify their survival or tumor burden.

### Example 4-URI Causes DNA Damage by Inhibiting De Novo NAD+ Synthesis

To identify initial hepatocarcinogenetic events responsible for DNA damage, the inventors first examined mTOR activation. They detected no increases in S6K1 activity at 1-week. In sequential immunoprecipitation experiments, using 1-week-old liver extracts, free hURI molecules were detected by WB after complete depletion of PP1γ, and vice versa. Furthermore, when 3-week-old mice were supplied a rapamycin-containing diet, progression to preneoplastic abnormalities continued. Thus, although a fraction of hURI binds PP1γ, hURI apparently has a PP1γ-independent role in early hepatocarcinogenesis and precancerous lesion formation.

The inventors therefore assessed unbiased global transcriptomic and proteomic profiles during a very early non-pathological stage with no signs of DNA damage or dysplastic foci and an early premalignant lesion stage, using 1- and 8-week-old livers, respectively. Transcript sequencing revealed that small fractions of genes were differentially expressed upon hURI expression: 303 out of 12,295 genes at 1 week and 740 out of 11,133 (FDR<0.05) at 8 weeks (Figures 4A and 4B). Similarly, isobaric Tags for Relative and Absolute Quantification (iTRAQ) identified 2394 proteins: 122 and 597 of which were differentially expressed in 1- and 8-week livers, respectively (Figures 4C and 4D).

Heatmapping revealed that most differentially expressed proteins were downregulated, indicating hURI-dependent repression mechanism. There were also significant overlaps in the differentially expressed transcripts and proteins at 1 and 8 weeks, suggesting that HURI may act as a transcriptional repressor. Ingenuity Pathway Analysis (IPA) revealed that among metabolic canonical pathways downregulated, the L-tryptophan/kynurenine catabolism leading to *de novo* nicotinamide adenine dinucleotide (NAD+) synthesis was one of the most significant downregulated pathway. All enzymes implicated in the L-tryptophan/kynurenine degradation, including tryptophan 2,3-dioxygenase (TDO2) and arylformamidase (AFMID) catalyzing the initial rate-limiting step of tryptophan degradation, kynurenine 3-monooxygenase (KMO), kynureninase (KNYU) and 3-hydroxyanthranilate 3,4-dioxygenase (HAAO) were strikingly downregulated in 1- and 8-week-old mutant livers (Figures 4E and 4F). Gene Set Enrichment Analysis (GSEA) using the RNA sequencing data and Kyoto Encyclopedia of Genes and Genomes database corroborated these defects (not shown). WB confirmed that TDO2 and AFMID expression was reduced >50% in these livers (Figures 4G and 4H). TDO2 and AFMID were also downregulated in adults expressing hURI, confirming an effect independent of embryonic liver development. Moreover, NAD+ concentrations were reduced in 3- and 6-week mutant livers, compared to controls (Figure 4I). In contrast, simultaneous increases in TDO2, AFMID and NAD+ levels were detected in URI(+/Δ)*hep* livers, indicating that URI reduction or deletion increases *de novo* NAD+ biosynthesis (Figures 4J and 4K). Furthermore, small interference RNA (siRNA) knockdown of TDO2 and AFMID in HCC cell lines significantly reduced NAD+ levels (Figures 9A and 9B). Finally, thin layer chromatography of metabolites after [14C]-tryptophan labeling in four human HCC cell lines (Huh7, HepG2, SNU398 and SNU449) highlighted the importance of *de novo* NAD+ synthesis in maintaining cellular NAD+ pools *in vivo.* Labeling in NAD+ was detected in all HCC cells, but markedly reduced following AFMID depletion (Figure 9C).

Consequently, enzymes involved in lipid synthesis were also predicted to be downregulated, as NAD+-dependent sirtuin activity acts a transcriptional regulator. Moreover, as shown later, the inventors demonstrated that hURI controlled activities of aryl hydrocarbon (AhR) and estrogen receptors (ER), transcribing enzymes of most of the top IPA-predicted downregulated metabolic pathways. Expression of key enzymes of three other pathways implicated in oncogenesis was unaffected by hURI expression: SHMT1, G6PD and GOT1 of the glycine/serine/threonine, pentose phosphate and glutamine/aspartate pathways, respectively (Figure 10A). Furthermore, expression of nicotinamide phosphoribosyltransferase (NAMPT) (implicated in NAD+ biosynthesis through salvage reactions) (Figure 10B) and activity of poly (ADP-ribose) polymerase (PARP), (the major NAD+-consuming enzyme) (Figure 10C) were not affected at an early stage (1 week) and levels of both NADH and several dehydrogenases that reduce NAD+ to NADH were all decreased (Figure 10D).

All these data suggest that reductions in NAD+ levels are mainly due to downregulation of L-tryptophan/kynurenine catabolism.

To validate the contribution of NAD+ to DNA integrity we used a chemical inhibitor, Ro-61-8048, of KMO, a key L-tryptophan catabolism enzyme (Figure 4F). The inventores first induced liver injury and hepatocyte proliferation by supplying C57BL/6 mice for 4 days with a DDC-supplemented diet. DMSO or Ro-61-8048 were intra-peritoneally injected during the next 3 days and mice were sacrificed on day 8. As expected, NAD+ concentrations were reduced and DNA damage foci significantly elevated in Ro-61-8048-treated livers (Figures 4L and 4M). Thus, L-tryptophan/kynurenine pathway inhibition leads to reduced NAD+ concentrations and DNA damage, recapitulating effects of hURI expression.

### Example 5-Restoring NAD+ Pools Protects from DNA Damage and Aberrant de novo NAD+ Synthesis is a Feature of Oncogenesis

In further experiments, the inventors asked whether restoring NAD+ pools at early times of hURI expression would prevent DNA damage associated-dysplastic nodule and tumor formation. Dietary supplementation of nicotinamide riboside (NR) has been described to replenish intracellular NAD+ levels *in vivo* (Canto et al., 2012 cited at supra). They supplied 3-week-old hURI-tetOFFhep mice a NR-supplemented diet. Without affecting their liver-to-body weight ratio, (Figure 11A) this significantly increased hepatic NAD+ concentrations relative to controls on chow (Figure 11B). We detected dysplastic lesions and DNA damage foci in all mutants on chow, but no dysplastic lesions and very few DNA damage foci, with reductions in fibrotic area and both p53 abundance and Ser-18 phosphorylation, in NR-supplemented mutants (Figures 5A-5F). Prolonged NR treatment also prevented tumor development in 30-week-old mice expressing hURI, which developed tumors when fed chow (Figure 5G; Figure 11C). Thus, restoring NAD+ pools protects from hURI-induced DNA damage, preneoplastic lesion formation-associated liver damage and tumor formation.

Furthermore, ceasing hURI expression in 8-week-old mice for 24 weeks restored AFMID levels, suppressed DNA damage, abolished the DDR and activated NAD+-dependent SIRT1 (manifested by reduced acetylation of p53 at Lys-379) (Figures 5H and 5I). Thus, continuous hURI expression and consequent inhibition of *de novo* NAD+ synthesis is essential for abolishing DNA repair and accelerating tumor formation.

Next, we explored whether other oncogenes had similar effects. *Ela-1-myc* mice, unlike *K-RASG12V* mice, develop pancreatic adenocarcinomas with high levels of DNA damage while pancreatic tumors initiated by K-RASG12V show no signs of replicative stress (Murga et al., 2011, Nat Struct Mol Biol 18, 1331-1335.). The inventors found that in early stages of tumorigenesis ectopic c-Myc expression induced DNA damage, but observed no γH2AX foci in pancreas from K-RasG12V mice (Figure 13A and 13B). The L-tryptophan/kynurenine pathway enzymes TOD2 and AFMID were clearly downregulated in *Ela-1-myc* but not K*-RasG12V* murine pancreas (Figure 13C), suggesting that oncogene-induced DNA damage may generally involve a metabolic reprogramming and NAD+ biosynthesis inhibition.

### Example 6-URI Regulates Kynurenine Metabolism by Modulating AhR and ER Activity

The inventors found significant overlaps in differentially expressed transcripts between their RNA sequencing and available microarray datasets for livers from *AhR-*/*-* and *ER-*/*-* mice (Matic et al., 2013, PLoS One 8, e57458; Tijet et al., 2006, Mol Pharmacol 69, 140-153) (Figures 6A and 6B), suggesting that AhR and ER mediate hURI-induced transcriptional repression of L-tryptophan/kynurenine catabolism. Furthermore, the enriched *ER*-/- sets included TDO2, corroborating reports that TDO2 is transcribed by ER. Accordingly, siRNA depletion of AhR and ER in HepG2 cells reduced expression of TDO2 (and AFMID), while URI downregulation increased their abundance (Figures 6C and 6D). ALGGEN-PROMO v3.0 software predicted several AhR and ER binding sites in genomic sequences 5 kb upstream of the transcriptional start sites of TDO2 and AFMID promoters. Chromatin immunoprecipitation assays using SNU449 cell extracts and PCR revealed that both AhR and ER bound to these promoters, at different sites, indicating that AhR and ER are indeed transcription factors of TDO2 and AFMID. The inventors also verified (by WB) hURI-induced downregulation of other AhR and ER target genes detected in the RNA sequencing and iTRAQ analyses, including carbomyl-phosphate synthase 1 (CPS1), glutayl-CoA dehydrogenase (GCDH) and glycine N-methyltransferase 1 (GNMT1)). Interestingly, *gnmt1-*/*-* mice develop chronic hepatitis and spontaneous HCC (Liao et al., 2009, Int J Cancer 124, 816-826). These data indicate that hURI may be a repressor of AhR and ER transcriptional factors transcribing several metabolic enzymes in particular implicated in tryptophan degradation to NAD+ synthesis.

AhR and ER were described to be in an inactive cytoplasmic complex with HSP90, a member of the URI prefoldin complex. Reciprocal co-immunoprecipitation experiments confirmed that hURI and HSP90 interact with AhR or ER in cytosolic extracts of 1-week-old mutant livers (Figures 6E and 6F). Furthermore, cytoplasmic fractions of mutant livers were enriched with both nuclear receptors (Figure 6G), indicating that hURI expression abolishes AhR and ER activity by inhibiting their cytoplasm-to-nucleus translocation. Confirmatory immunofluorescence analysis detected significant reductions in nuclear AhR and ER in hepatocytes of 1-week-old mutants (Figures 6H and 6I). Thus, AhR and ER are apparently trapped in an inhibitory cytoplasmic complex containing hURI and HSP90, preventing their trafficking to the nucleus and inhibiting transcription of L-tryptophan/kynurenine catabolic enzymes.

### Example 7-URI Expression Is Enhanced in Human HCC and Correlates with De Novo NAD+ Synthesis Inhibition

To evaluate URI's relevance in human hepatocarcinogenesis, the inventors examined by IHC its expression in a tissue-microarray (TMA) of 49 human liver samples (36 HCC, 4 peritumoral and 9 normal liver). Using a specific anti-URI antibody, they detected no, weak and strong URI expression in normal livers, peritumoral areas of HCC patients and 60% of HCCs, respectively (Figures 7A and 7B). Increased URI levels in 20 tumoral human samples, relative to paired peritumoral samples, were also detected by real-time PCR and WB, with a positive correlation between URI expression and tumor aggressiveness, detected by Ki67 staining (Figure 7C). URI expression was approximately twice as strong in 70% of the tumoral tissues as in peritumoral counterparts, corresponding to the hURI expression level in the hURI-tetOFFhep mouse. Stratification of the data indicated a significant correlation between URI expression and HBV- or HCV-associated HCC (Figure 7D). Increased URI expression was therefore observed in human hepatitis samples, a predisposed stage of hepatocarcinogenesis (Figures 7E and 7F). Importantly, GSEA detected significant overlaps between transcriptomic signatures of our hURI GEMM and HBV-associated human HCC (Huang et al., 2011, PLoS One 6, e26168.) (Figures 7G and 7H).

Next, the inventors examined correlations between URI expression and L-tryptophan/kynurenine pathway enzymes in human HCC. WB analysis of the paired peritumoral and tumoral (HCC) samples revealed that AFMID and NAD+ levels were positively correlated, and both negatively correlated with URI expression. Comparative analysis using a human HCC gene array dataset (Wurmbach et al., 2007, Hepatology *45*, 938-947) detected inverse correlations between URI expression and TDO2, KMO and HAAO expression (Figure 7I). Moreover, using a database for 221 patients (Roessler et al., 2010, Cancer Res 70, 10202-10212) (GEO ID: GSE14520), Cox regression analysis of TDO2, KMO, HAOO and quinolinate phosphoribosyltransferase (QPRT) expression indicated that the patients overall survival was significantly associated (p = 0.025) with our tryptophan catabolism signature (Figure 7J). Downregulation of TDO2 or HAAO also predicted poor prognosis for HCC patients. Thus, URI-mediated *de novo* NAD+ synthesis inhibition occurs in human HCC development.

## Claims

1. Nicotinamide riboside or a derivative thereof for use in the treatment and/or prevention of liver cancer or pancreatic cancer in a subject.

2. Nicotinamide riboside or a derivative thereof for use according to claim 1, wherein the subject shows high level of expression of a gene inducing DNA damage.

3. Nicotinamide riboside or a derivative thereof for use according to claim 2, wherein the gene inducing DNA damage is selected from the group consisting of URI gene and c-Myc gene.

4. Nicotinamide riboside or a derivative thereof for use according to any of claims 1-3, wherein the liver cancer is hepatocellular carcinoma and the pancreatic cancer is pancreatic ductal adenocarcinoma.

5. Nicotinamide riboside or a derivative thereof for use according to any of claims 1 to 4, wherein the subject shows early signs of chronic liver damage or pancreatic intraepithelial lesions.

6. Nicotinamide riboside or a derivative thereof for use according to any of claims 1 to 5, wherein the nicotinamide riboside or a derivative thereof is administered in combination with a chemotherapeutic agent.

7. Nicotinamide riboside or a derivative thereof for use according to claim 6, wherein the chemotherapeutic agent is selected from Table 1.

8. An *in vitro* method for designing a customized therapy for a subject diagnosed with a liver cancer or pancreatic cancer, suffering early signs of chronic liver damage or suffering pancreatic intraepithelial lesions which comprises
a) determining the level of DNA damage in a sample from said subject, and
b) comparing said levels with a reference value,
wherein an increased level of DNA damage in said sample with respect to the reference value is indicative that the subject is to be treated with nicotinamide riboside or a derivative thereof.

9. An *in vitro* method according to claim 8 wherein the gene inducing DNA damage is selected from URI and c-Myc.

10. An *in vitro* method according to any of claims 8 or 9, wherein the liver cancer is hepatocellular carcinoma and the pancreatic cancer is pancreatic ductal adenocarcinoma.

11. An *in vitro* method for diagnosing liver cancer in a subject which comprises:
a) determining the level of expression of URI gene in a sample from said subject, and
b) comparing said level with a reference value,
wherein an increased level of expression of URI gene in said sample with respect to the reference value is indicative that the subject suffers liver cancer.

12. An *in vitro* method for predicting the risk of developing liver cancer in a subject suffering hepatitis which comprises:
a) determining the level of expression of URI gene in a sample from said subject, and
b) comparing said levels with a reference value,
wherein an increased level of expression of URI gene in said sample with respect to the reference value is indicative that the subject shows high risk of developing liver cancer.

13. Kit comprising a reagent which allows determining the expression level of URI gene.

14. Kit according to claim 13, further comprising a reagent which allows determining the NAD+ levels.

15. Use of a kit according to any of claims 13 or 14 for designing a customized therapy for a subject diagnosed with a liver cancer or suffering early signs of chronic liver damage, for diagnosing liver cancer or for predicting the risk of a patient suffering hepatitis to develop liver cancer.
